# EUROPEAN PATENT APPLICATION

(11) **EP 4 382 618 A1**
(43) Date of publication of application: **12.06.2024**
(21) Application number: 22852987.1
(22) Date of filing: 01.08.2022
(51) Int. Cl.: C12Q 1/6886, C12N 15/09, C12Q 1/6813

(54) **METHOD FOR DIAGNOSING CANINE CANCER**

(30) Priority: 02.08.2021 JP 2021126589
(71) Applicant: Medical Ark, Inc., Mitaka-shi, Tokyo1810013 (JP)
(72) Inventor: OCHIYA Takahiro, Tokyo 160-0023 (JP); ITOH Hiroshi, Mitaka-shi, Tokyo 181-0013 (JP); TSUCHIYA Reiko, Tokyo 113-0033 (JP)
(74) Representative: Zacco GmbH
(86) International application number: PCT/JP2022/029420
(87) International publication number: WO 2023/013568

(57) **Abstract**

Provided is a method for diagnosing canine cancer involving making a computer diagnose based on the results of the gene expression analysis using dog microRNA. A method for diagnosing canine cancer, comprising: a diagnostic step of acquiring the result of the gene expression analysis of microRNA extracted from body fluid of a dog to be diagnosed, determining the gene expression level of a specific microRNA using the result, and diagnosing the degree of a risk of the dog to be diagnosed being suffering from a specific cancer disease using the gene expression level as a diagnostic criterion, characterized in that the specific microRNA is a microRNA showing a significant difference in the gene expression level between a dog suffering from the specific cancer disease and a healthy dog based on the result of the gene expression analysis of the microRNA extracted from the body fluid of the dog.

## Description

### Technical Field

The present invention relates to a method for diagnosing canine cancer based on the results of the gene expression analysis of a microRNA.

### Background Art

In cancer treatment, early detection and early treatment are important. A problem was, however, that the conventional tests were invasive methods such as the pathological tests of excised tumor tissue, and imposed great burdens on patients, and it was difficult to repeat the conventional tests. Thereupon, it is a technique for detecting cancer by analyzing a specific biomarker derived from body fluid such as blood, saliva, or urine, namely a liquid biopsy, that has been attracting attention in recent years. Such a technique has the advantage that the technique imposes a light burden on patients, body fluid can be repeatedly collected, and not only a single organ but also the whole body can be furthermore screened. It is believed that the technique is useful not only for early diagnosis of cancer but also for posttreatment recurrence monitoring.

It is known that a microRNA is a small RNA having around 22 bases and contained in body fluid such as blood, saliva, or urine, and controls specific gene expression, and the type and amount thereof vary in blood of a patient with disease such as cancer. The microRNA is used as a cancer-specific biomarker in humans, and the development of a kit for diagnosing cancer based on the results of the expression analysis thereof has been advanced (for example, Patent Literature 1).

However, an effective technique for detecting cancer targeted at dogs has yet to be established.

### Document List

### Patent Literature

Patent Literature 1: WO 2015/194615 A1

### Summary of Invention

### Technical Problem

The present invention provides an accurate and rapid method for diagnosing canine cancer based on the results of the gene expression analysis using a dog microRNA.

### Solution to Problem

Embodiments of the present invention are as follows.
[1] A method for diagnosing canine cancer, comprising: a diagnostic step of acquiring a result of gene expression analysis of microRNA extracted from body fluid of a dog to be diagnosed, determining a gene expression level of a specific microRNA using the result, and diagnosing a degree of a risk of the dog to be diagnosed being suffering from a specific cancer disease using the gene expression level as a diagnostic criterion, characterized in that the specific microRNA is a microRNA showing a significant difference in gene expression level between a dog suffering from the specific cancer disease and a healthy dog based on the result of the gene expression analysis of the microRNA extracted from the body fluid of the dog.
[2] The method according to [1], further comprising a step of sampling the microRNA showing a significant difference in gene expression level between the dog suffering from the specific cancer disease and the healthy dog based on the result of the gene expression analysis of the microRNA extracted from the body fluid of the dog as the specific microRNA on the basis of a discriminant created using statistical analysis processing before the diagnostic step.
[3] The method according to [1] or [2], wherein the specific cancer disease is selected from the group consisting of intraoral melanoma, urothelial cancer, malignant lymphoma, hepatocellular cancer, and mastocytoma.
[4] The method according to any of [1] to [3], wherein the specific cancer disease is five cancer diseases including intraoral melanoma, urothelial cancer, malignant lymphoma, hepatocellular cancer, and mastocytoma, and the specific microRNA is at least one selected from the group consisting of cfa-let-7c, cfa-miR-103, cfa-miR-10a, cfa-miR-122, cfa-miR-125a, cfa-miR-125b, cfa-miR-126, cfa-miR-130a, cfa-miR-144, cfa-miR-150, cfa-miR-155, cfa-miR-186, cfa-miR-193a, cfa-miR-197, cfa-miR-199, cfa-miR-19b, cfa-miR-22, cfa-miR-222, cfa-miR-223, cfa-miR-24, cfa-miR-26a, cfa-miR-27b, cfa-miR-339, cfa-miR-342, cfa-miR-378, cfa-miR-383, cfa-miR-483, cfa-miR-486-3p, cfa-miR-489, cfa-miR-551b, cfa-miR-660, cfa-miR-718, cfa-miR-874, cfa-miR-8794, cfa-miR-8798, cfa-miR-8843, cfa-miR-8859a, cfa-miR-8860, cfa-miR-8903, cfa-miR-8906, cfa-miR-8907, cfa-miR-8908a-3p, cfa-miR-8908d and cfa-miR-92b.
[5] The method according to any of [1] to [3], wherein the specific cancer disease is intraoral melanoma, and the specific microRNA is at least one selected from the group consisting of cfa-miR-10a, cfa-miR-1185, cfa-miR-125a, cfa-miR-126, cfa-miR-144, cfa-miR-146a, cfa-miR-149, cfa-miR-150, cfa-miR-155, cfa-miR-184, cfa-miR-186, cfa-miR-197, cfa-miR-199, cfa-miR-19b, cfa-miR-24, cfa-miR-30c, cfa-miR-483, cfa-miR-489, cfa-miR-8798, cfa-miR-8816, cfa-miR-8875 and cfa-miR-8908a-3p.
[6] The method according to any of [1] to [3], wherein the specific cancer disease is urothelial cancer, and the specific microRNA is at least one selected from the group consisting of cfa-let-7f, cfa-miR-10a, cfa-miR-1249, cfa-miR-125a, cfa-miR-126, cfa-miR-1306, cfa-miR-130a, cfa-miR-133b, cfa-miR-144, cfa-miR-146a, cfa-miR-149, cfa-miR-150, cfa-miR-155, cfa-miR-1844, cfa-miR-186, cfa-miR-193a, cfa-miR-197, cfa-miR-19b, cfa-miR-2114, cfa-miR-24, cfa-miR-29b, cfa-miR-339, cfa-miR-342, cfa-miR-361, cfa-miR-370, cfa-miR-425, cfa-miR-483, cfa-miR-494, cfa-miR-502, cfa-miR-551b, cfa-miR-660, cfa-miR-665, cfa-miR-718, cfa-miR-8798, cfa-miR-8824, cfa-miR-8832, cfa-miR-8834a, cfa-miR-8843, cfa-miR-8904b and cfa-miR-8907, cfa-miR-8908a-3p.
[7] The method according to any of [1] to [3], wherein the specific cancer disease is malignant lymphoma, and the specific microRNA is at least one selected from the group consisting of cfa-miR-1185, cfa-miR-122, cfa-miR-1249, cfa-miR-126, cfa-miR-1306, cfa-miR-144, cfa-miR-150, cfa-miR-184, cfa-miR-188, cfa-miR-197, cfa-miR-199, cfa-miR-24, cfa-miR-342, cfa-miR-345, cfa-miR-378, cfa-miR-425, cfa-miR-574, cfa-miR-8794, cfa-miR-8797, cfa-miR-8798, cfa-miR-8900, cfa-miR-8904b, cfa-miR-8907 and cfa-miR-8908d.
[8] The method according to any of [1] to [3], wherein the specific cancer disease is hepatocellular cancer, and the specific microRNA is at least one selected from the group consisting of cfa-let-7g, cfa-miR-10a, cfa-miR-1185, cfa-miR-122, cfa-miR-125b, cfa-miR-150, cfa-miR-155, cfa-miR-197, cfa-miR-199, cfa-miR-206, cfa-miR-22, cfa-miR-378, cfa-miR-383, cfa-miR-483, cfa-miR-665, cfa-miR-7, cfa-miR-718, cfa-miR-874, cfa-miR-8794, cfa-miR-8798, cfa-miR-8875, cfa-miR-8900, cfa-miR-8902, cfa-miR-8907, cfa-miR-8908a-3p, cfa-miR-8908d, cfa-miR-92b and cfa-miR-99a.
[9] The method according to any of [1] to [3], wherein the specific cancer disease is mastocytoma, and the specific microRNA is at least one selected from the group consisting of cfa-miR-10b, cfa-miR-1185, cfa-miR-125b, cfa-miR-126, cfa-miR-149, cfa-miR-150, cfa-miR-155, cfa-miR-197, cfa-miR-199, cfa-miR-222, cfa-miR-24, cfa-miR-26b, cfa-miR-8803, cfa-miR-8872, cfa-miR-8903, cfa-miR-8907 and cfa-miR-92b.
[10] A method for diagnosing canine cancer, comprising: a diagnostic step of acquiring a result of gene expression analysis of microRNA extracted from body fluid of a dog to be diagnosed, determining a gene expression level of a specific microRNA using the result, and diagnosing a degree of a risk of the dog to be diagnosed being suffering from a specific cancer disease using the gene expression level as a diagnostic criterion, characterized in that the specific microRNA is a microRNA showing a significant difference in gene expression level among a dog suffering from the specific cancer disease, a dog suffering from another cancer disease, and a healthy dog based on the result of the gene expression analysis of the microRNA extracted from the body fluid of the dog.
[11] The method according to [10], further comprising a step of sampling the microRNA showing a significant difference in gene expression level among the dog suffering from the specific cancer disease, the dog suffering from other cancer disease, and the healthy dog based on the result of the gene expression analysis of the microRNA extracted from the body fluid of the dog as the specific microRNA on the basis of a discriminant created using statistical analysis processing before the diagnostic step.
[12] The method according to [10], comprising: the degree of the risk of the dog to be diagnosed being suffering from the specific cancer disease being higher or lower than a risk of being suffering from other cancer disease in the diagnostic step.
[13] The method according to any of [10] to [12], wherein the specific cancer disease is selected from the group consisting of intraoral melanoma, urothelial cancer, malignant lymphoma, hepatocellular cancer, and mastocytoma.
[14] The method according to any of [10] to [13], wherein the specific cancer disease is intraoral melanoma, and the specific microRNA that is used in the diagnosis is at least one selected from the group consisting of cfa-miR-1185, cfa-miR-122, cfa-miR-126, cfa-miR-1271, cfa-miR-1306, cfa-miR-130b, cfa-miR-140, cfa-miR-144, cfa-miR-146a, cfa-miR-150, cfa-miR-155, cfa-miR-192, cfa-miR-197, cfa-miR-222, cfa-miR-29a, cfa-miR-29b, cfa-miR-30b, cfa-miR-370, cfa-miR-378, cfa-miR-486, cfa-miR-486-3p, cfa-miR-489, cfa-miR-494, cfa-miR-532, cfa-miR-551b, cfa-miR-660, cfa-miR-8803, cfa-miR-8816, cfa-miR-8824, cfa-miR-8834b, cfa-miR-8859a, cfa-miR-8875, cfa-miR-8907, cfa-miR-8908d and cfa-miR-92b.
[15] The method according to any of [10] to [13], wherein the specific cancer disease is urothelial cancer, and the specific microRNA that is used in the diagnosis is at least one selected from the group consisting of cfa-let-7b, cfa-miR-107, cfa-miR-10b, cfa-miR-122, cfa-miR-125b, cfa-miR-1306, cfa-miR-130a, cfa-miR-133b, cfa-miR-144, cfa-miR-146a, cfa-miR-149, cfa-miR-181a, cfa-miR-185, cfa-miR-193a, cfa-miR-197, cfa-miR-19b, cfa-miR-23a, cfa-miR-29c, cfa-miR-345, cfa-miR-361, cfa-miR-370, cfa-miR-378, cfa-miR-425, cfa-miR-486, cfa-miR-486-3p, cfa-miR-494, cfa-miR-532, cfa-miR-551b, cfa-miR-660, cfa-miR-8794, cfa-miR-8803, cfa-miR-8816, cfa-miR-8834b, cfa-miR-8860, cfa-miR-8873a, cfa-miR-8891, cfa-miR-8903, cfa-miR-8904b, cfa-miR-8907, cfa-miR-8908d, cfa-miR-92b, cfa-miR-93 and cfa-miR-99a.
[16] The method according to any of [10] to [13], wherein the specific cancer disease is malignant lymphoma, and the specific microRNA that is used in the diagnosis is at least one selected from the group consisting of cfa-let-7f, cfa-miR-122, cfa-miR-1306, cfa-miR-130a, cfa-miR-140, cfa-miR-144, cfa-miR-146a, cfa-miR-151, cfa-miR-15a, cfa-miR-181a, cfa-miR-1844, cfa-miR-188, cfa-miR-192, cfa-miR-193a, cfa-miR-21, cfa-miR-22, cfa-miR-23b, cfa-miR-24, cfa-miR-26a, cfa-miR-26b, cfa-miR-27b, cfa-miR-301a, cfa-miR-339, cfa-miR-342, cfa-miR-345, cfa-miR-378, cfa-miR-486, cfa-miR-486-3p, cfa-miR-551b, cfa-miR-574, cfa-miR-631, cfa-miR-8794, cfa-miR-8803, cfa-miR-8809, cfa-miR-8836, cfa-miR-8843, cfa-miR-8863, cfa-miR-8872, cfa-miR-8904b, cfa-miR-8906, cfa-miR-8907 and cfa-miR-8908d.
[17] The method according to any of [10] to [13], wherein the specific cancer disease is hepatocellular cancer, and the specific microRNA that is used in the diagnosis is at least one selected from the group consisting of cfa-let-7b, cfa-let-7c, cfa-let-7f, cfa-let-7g, cfa-miR-10b, cfa-miR-122, cfa-miR-1249, cfa-miR-132, cfa-miR-134, cfa-miR-146a, cfa-miR-150, cfa-miR-155, cfa-miR-16, cfa-miR-206, cfa-miR-22, cfa-miR-223, cfa-miR-29a, cfa-miR-30d, cfa-miR-331, cfa-miR-378, cfa-miR-425, cfa-miR-483, cfa-miR-486-3p, cfa-miR-494, cfa-miR-532, cfa-miR-574, cfa-miR-8794, cfa-miR-8815, cfa-miR-8824, cfa-miR-8834b, cfa-miR-8836, cfa-miR-8843, cfa-miR-8860, cfa-miR-8892, cfa-miR-8900, cfa-miR-8903, cfa-miR-8908a-3p, cfa-miR-8908d, cfa-miR-92b and cfa-miR-99a.
[18] The method according to any of [10] to [13], wherein the specific cancer disease is mastocytoma, and the specific microRNA that is used in the diagnosis is at least one selected from the group consisting of cfa-let-7c, cfa-let-7f, cfa-miR-10a, cfa-miR-1306, cfa-miR-130a, cfa-miR-144, cfa-miR-149, cfa-miR-186, cfa-miR-188, cfa-miR-18a, cfa-miR-191, cfa-miR-221, cfa-miR-223, cfa-miR-301a, cfa-miR-30c, cfa-miR-342, cfa-miR-370, cfa-miR-451, cfa-miR-486-3p, cfa-miR-551b, cfa-miR-574, cfa-miR-631, cfa-miR-8809, cfa-miR-8815, cfa-miR-8816, cfa-miR-8834a, cfa-miR-8834b, cfa-miR-8903 and cfa-miR-92b.
[19] A biomarker for diagnosing five canine cancer diseases including intraoral melanoma, urothelial cancer, malignant lymphoma, hepatocellular cancer, and mastocytoma, comprising: at least one microRNA selected from the group consisting of cfa-let-7c, cfa-miR-103, cfa-miR-10a, cfa-miR-122, cfa-miR-125a, cfa-miR-125b, cfa-miR-126, cfa-miR-130a, cfa-miR-144, cfa-miR-150, cfa-miR-155, cfa-miR-186, cfa-miR-193a, cfa-miR-197, cfa-miR-199, cfa-miR-19b, cfa-miR-22, cfa-miR-222, cfa-miR-223, cfa-miR-24, cfa-miR-26a, cfa-miR-27b, cfa-miR-339, cfa-miR-342, cfa-miR-378, cfa-miR-383, cfa-miR-483, cfa-miR-486-3p, cfa-miR-489, cfa-miR-551b, cfa-miR-660, cfa-miR-718, cfa-miR-874, cfa-miR-8794, cfa-miR-8798, cfa-miR-8843, cfa-miR-8859a, cfa-miR-8860, cfa-miR-8903, cfa-miR-8906, cfa-miR-8907, cfa-miR-8908a-3p, cfa-miR-8908d and cfa-miR-92b.
[20] A biomarker for diagnosing dog intraoral melanoma, comprising: at least one of microRNA selected from the group consisting of cfa-miR-10a, cfa-miR-1185, cfa-miR-125a, cfa-miR-126, cfa-miR-144, cfa-miR-146a, cfa-miR-149, cfa-miR-150, cfa-miR-155, cfa-miR-184, cfa-miR-186, cfa-miR-197, cfa-miR-199, cfa-miR-19b, cfa-miR-24, cfa-miR-30c, cfa-miR-483, cfa-miR-489, cfa-miR-8798, cfa-miR-8816, cfa-miR-8875 and cfa-miR-8908a-3p.
[21] A biomarker for diagnosing dog urothelial cancer, comprising: at least one microRNA selected from the group consisting of cfa-let-7f, cfa-miR-10a, cfa-miR-1249, cfa-miR-125a, cfa-miR-126, cfa-miR-1306, cfa-miR-130a, cfa-miR-133b, cfa-miR-144, cfa-miR-146a, cfa-miR-149, cfa-miR-150, cfa-miR-155, cfa-miR-1844, cfa-miR-186, cfa-miR-193a, cfa-miR-197, cfa-miR-19b, cfa-miR-2114, cfa-miR-24, cfa-miR-29b, cfa-miR-339, cfa-miR-342, cfa-miR-361, cfa-miR-370, cfa-miR-425, cfa-miR-483, cfa-miR-494, cfa-miR-502, cfa-miR-551b, cfa-miR-660, cfa-miR-665, cfa-miR-718, cfa-miR-8798, cfa-miR-8824, cfa-miR-8832, cfa-miR-8834a, cfa-miR-8843, cfa-miR-8904b and cfa-miR-8907, cfa-miR-8908a-3p.
[22] A biomarker for diagnosing dog malignant lymphoma, comprising: at least one microRNA selected from the group consisting of cfa-miR-1185, cfa-miR-122, cfa-miR-1249, cfa-miR-126, cfa-miR-1306, cfa-miR-144, cfa-miR-150, cfa-miR-184, cfa-miR-188, cfa-miR-197, cfa-miR-199, cfa-miR-24, cfa-miR-342, cfa-miR-345, cfa-miR-378, cfa-miR-425, cfa-miR-574, cfa-miR-8794, cfa-miR-8797, cfa-miR-8798, cfa-miR-8900, cfa-miR-8904b, cfa-miR-8907 and cfa-miR-8908d.
[23] A biomarker for diagnosing dog hepatocellular cancer, comprising: at least one microRNA selected from the group consisting of cfa-let-7g, cfa-miR-10a, cfa-miR-1185, cfa-miR-122, cfa-miR-125b, cfa-miR-150, cfa-miR-155, cfa-miR-197, cfa-miR-199, cfa-miR-206, cfa-miR-22, cfa-miR-378, cfa-miR-383, cfa-miR-483, cfa-miR-665, cfa-miR-7, cfa-miR-718, cfa-miR-874, cfa-miR-8794, cfa-miR-8798, cfa-miR-8875, cfa-miR-8900, cfa-miR-8902, cfa-miR-8907, cfa-miR-8908a-3p, cfa-miR-8908d, cfa-miR-92b and cfa-miR-99a.
[24] A biomarker for diagnosing dog mastocytoma, comprising: at least one microRNA selected from the group consisting of cfa-miR-10b, cfa-miR-1185, cfa-miR-125b, cfa-miR-126, cfa-miR-149, cfa-miR-150, cfa-miR-155, cfa-miR-197, cfa-miR-199, cfa-miR-222, cfa-miR-24, cfa-miR-26b, cfa-miR-8803, cfa-miR-8872, cfa-miR-8903, cfa-miR-8907 and cfa-miR-92b.
[25] A biomarker for diagnosing dog intraoral melanoma, comprising: at least one microRNA selected from the group consisting of cfa-miR-1185, cfa-miR-122, cfa-miR-126, cfa-miR-1271, cfa-miR-1306, cfa-miR-130b, cfa-miR-140, cfa-miR-144, cfa-miR-146a, cfa-miR-150, cfa-miR-155, cfa-miR-192, cfa-miR-197, cfa-miR-222, cfa-miR-29a, cfa-miR-29b, cfa-miR-30b, cfa-miR-370, cfa-miR-378, cfa-miR-486, cfa-miR-486-3p, cfa-miR-489, cfa-miR-494, cfa-miR-532, cfa-miR-551b, cfa-miR-660, cfa-miR-8803, cfa-miR-8816, cfa-miR-8824, cfa-miR-8834b, cfa-miR-8859a, cfa-miR-8875, cfa-miR-8907, cfa-miR-8908d and cfa-miR-92b.
[26] A biomarker for diagnosing dog urothelial cancer, comprising: at least one microRNA selected from the group consisting of cfa-let-7b, cfa-miR-107, cfa-miR-10b, cfa-miR-122, cfa-miR-125b, cfa-miR-1306, cfa-miR-130a, cfa-miR-133b, cfa-miR-144, cfa-miR-146a, cfa-miR-149, cfa-miR-181a, cfa-miR-185, cfa-miR-193a, cfa-miR-197, cfa-miR-19b, cfa-miR-23a, cfa-miR-29c, cfa-miR-345, cfa-miR-361, cfa-miR-370, cfa-miR-378, cfa-miR-425, cfa-miR-486, cfa-miR-486-3p, cfa-miR-494, cfa-miR-532, cfa-miR-551b, cfa-miR-660, cfa-miR-8794, cfa-miR-8803, cfa-miR-8816, cfa-miR-8834b, cfa-miR-8860, cfa-miR-8873a, cfa-miR-8891, cfa-miR-8903, cfa-miR-8904b, cfa-miR-8907, cfa-miR-8908d, cfa-miR-92b, cfa-miR-93 and cfa-miR-99a.
[27] A biomarker for diagnosing dog malignant lymphoma, comprising: at least one microRNA selected from the group consisting of cfa-let-7f, cfa-miR-122, cfa-miR-1306, cfa-miR-130a, cfa-miR-140, cfa-miR-144, cfa-miR-146a, cfa-miR-151, cfa-miR-15a, cfa-miR-181a, cfa-miR-1844, cfa-miR-188, cfa-miR-192, cfa-miR-193a, cfa-miR-21, cfa-miR-22, cfa-miR-23b, cfa-miR-24, cfa-miR-26a, cfa-miR-26b, cfa-miR-27b, cfa-miR-301a, cfa-miR-339, cfa-miR-342, cfa-miR-345, cfa-miR-378, cfa-miR-486, cfa-miR-486-3p, cfa-miR-551b, cfa-miR-574, cfa-miR-631, cfa-miR-8794, cfa-miR-8803, cfa-miR-8809, cfa-miR-8836, cfa-miR-8843, cfa-miR-8863, cfa-miR-8872, cfa-miR-8904b, cfa-miR-8906, cfa-miR-8907 and cfa-miR-8908d.
[28] A biomarker for diagnosing dog hepatocellular cancer, comprising: at least one microRNA selected from the group consisting of cfa-let-7b, cfa-let-7c, cfa-let-7f, cfa-let-7g, cfa-miR-10b, cfa-miR-122, cfa-miR-1249, cfa-miR-132, cfa-miR-134, cfa-miR-146a, cfa-miR-150, cfa-miR-155, cfa-miR-16, cfa-miR-206, cfa-miR-22, cfa-miR-223, cfa-miR-29a, cfa-miR-30d, cfa-miR-331, cfa-miR-378, cfa-miR-425, cfa-miR-483, cfa-miR-486-3p, cfa-miR-494, cfa-miR-532, cfa-miR-574, cfa-miR-8794, cfa-miR-8815, cfa-miR-8824, cfa-miR-8834b, cfa-miR-8836, cfa-miR-8843, cfa-miR-8860, cfa-miR-8892, cfa-miR-8900, cfa-miR-8903, cfa-miR-8908a-3p, cfa-miR-8908d, cfa-miR-92b and cfa-miR-99a.
[29] A biomarker for diagnosing dog mastocytoma, comprising: at least one microRNA selected from the group consisting of cfa-let-7c, cfa-let-7f, cfa-miR-10a, cfa-miR-1306, cfa-miR-130a, cfa-miR-144, cfa-miR-149, cfa-miR-186, cfa-miR-188, cfa-miR-18a, cfa-miR-191, cfa-miR-221, cfa-miR-223, cfa-miR-301a, cfa-miR-30c, cfa-miR-342, cfa-miR-370, cfa-miR-451, cfa-miR-486-3p, cfa-miR-551b, cfa-miR-574, cfa-miR-631, cfa-miR-8809, cfa-miR-8815, cfa-miR-8816, cfa-miR-8834a, cfa-miR-8834b, cfa-miR-8903 and cfa-miR-92b.

### Effects of Invention

An accurate and rapid method for diagnosing canine cancer is provided by the present invention.

### Brief Description of Drawings

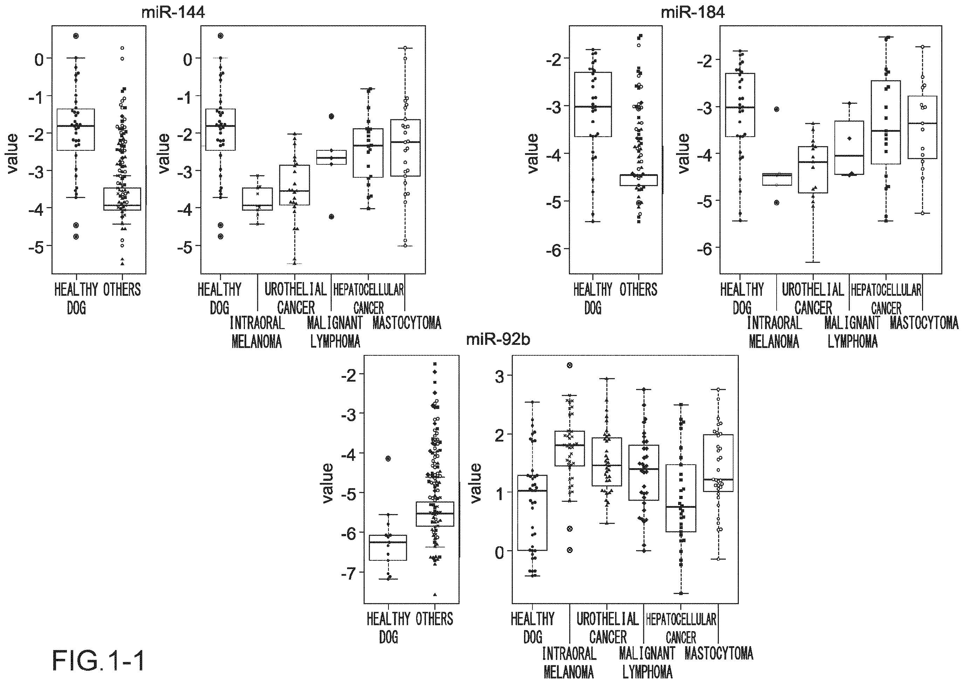

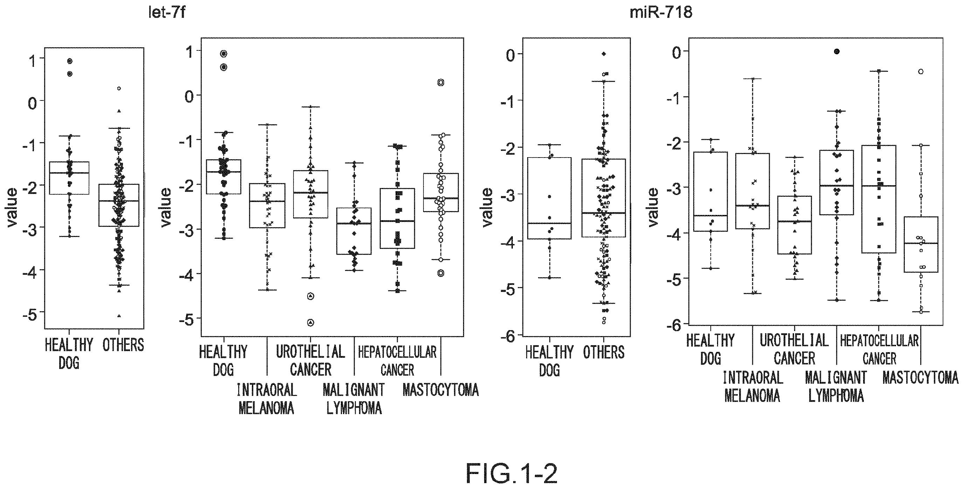

[FIG. 1-1] Figures showing the results of the gene expression analysis of microRNAs.
[FIG. 1-2] Figures showing the results of the gene expression analysis of microRNAs.
[FIG. 2] Figures showing the ROC curves and the AUCs of model discriminants.
[FIG. 3] Figures showing the ROC curves and the AUCs of model discriminants.
[FIG. 4-1] Figures showing discriminants sampled in the implementation of the present invention and the AUCs thereof.
[FIG. 4-2] Figures showing discriminants sampled in the implementation of the present invention and the AUCs thereof.
[FIG. 4-3] Figures showing discriminants sampled in the implementation of the present invention and the AUCs thereof.
[FIG. 4-4] Figures showing discriminants sampled in the implementation of the present invention and the AUCs thereof.

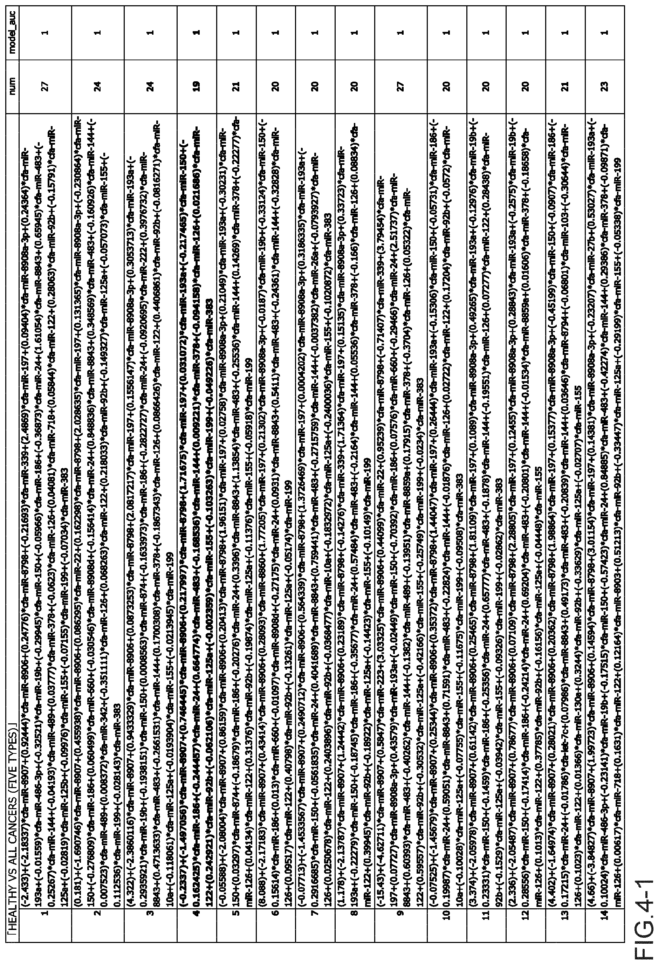

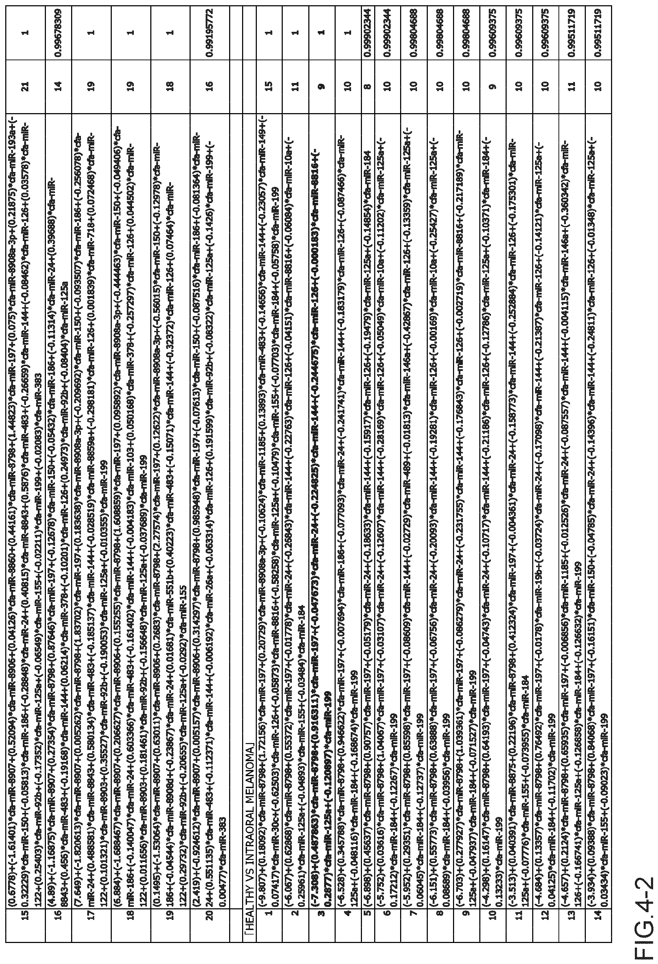

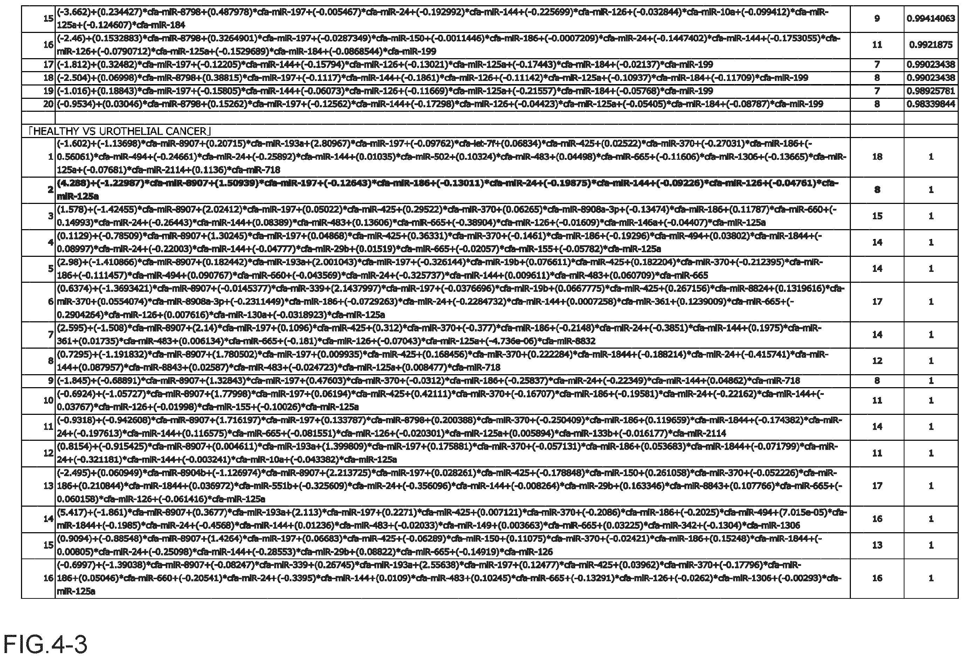

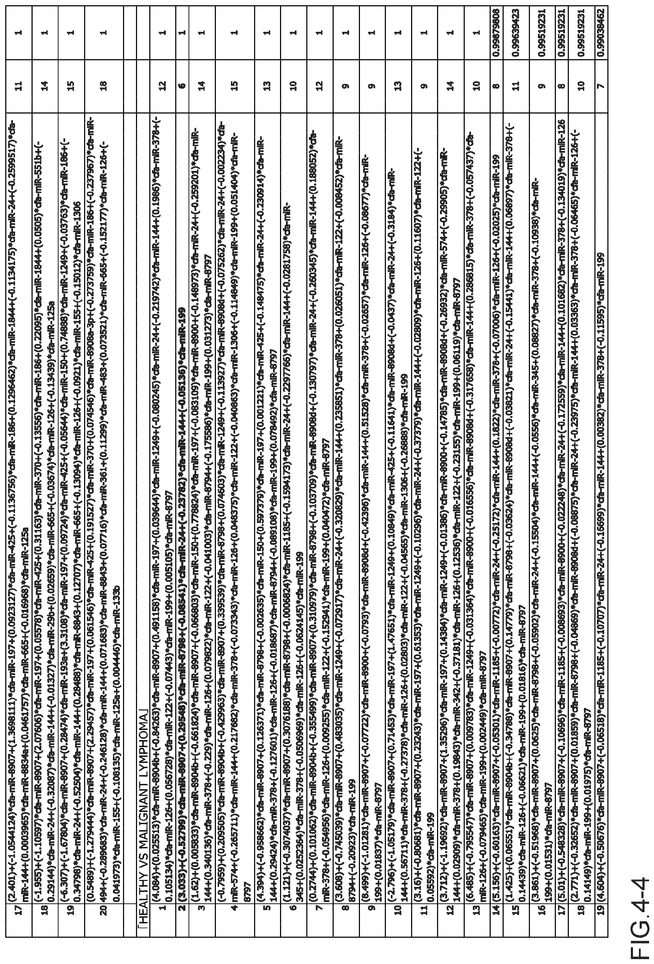

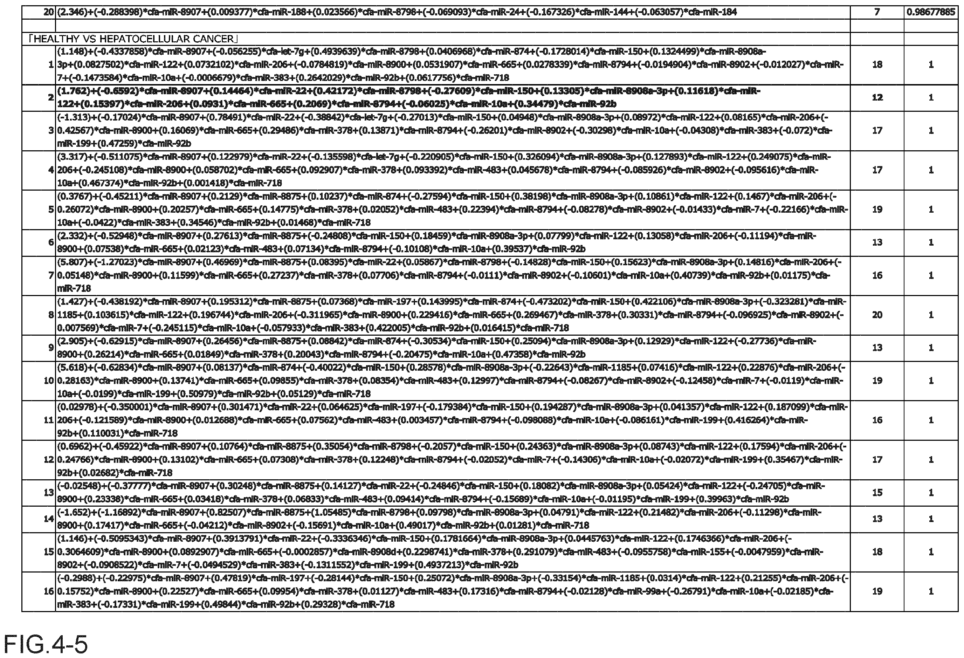

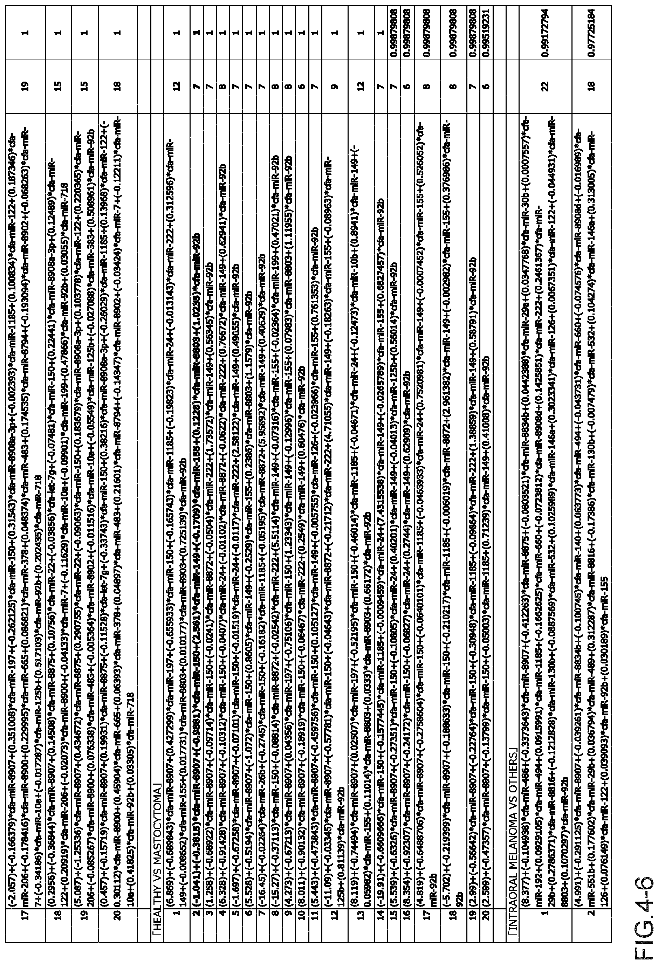

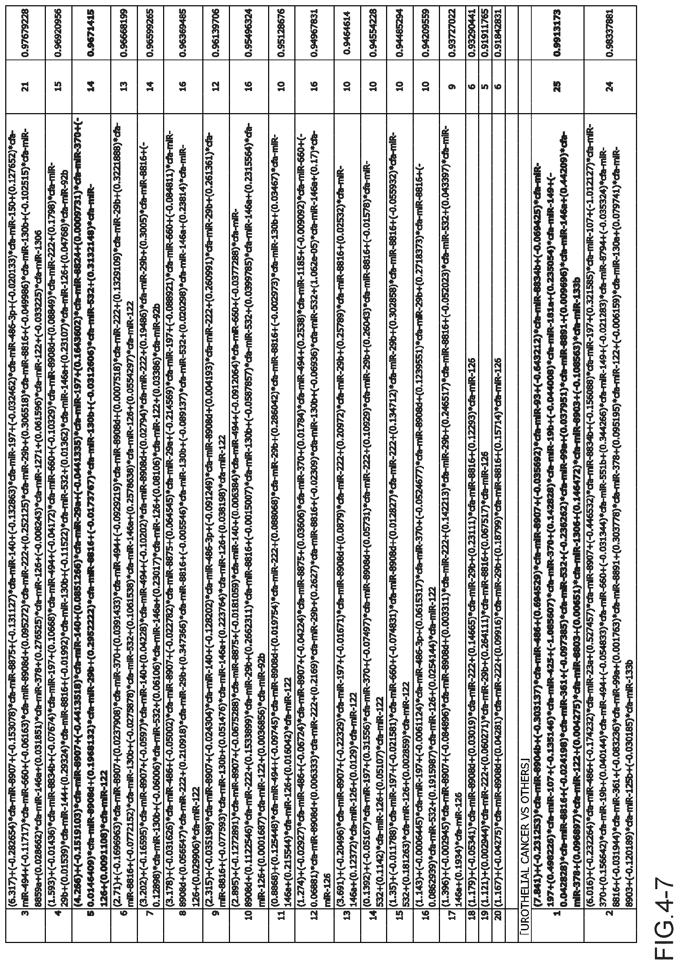

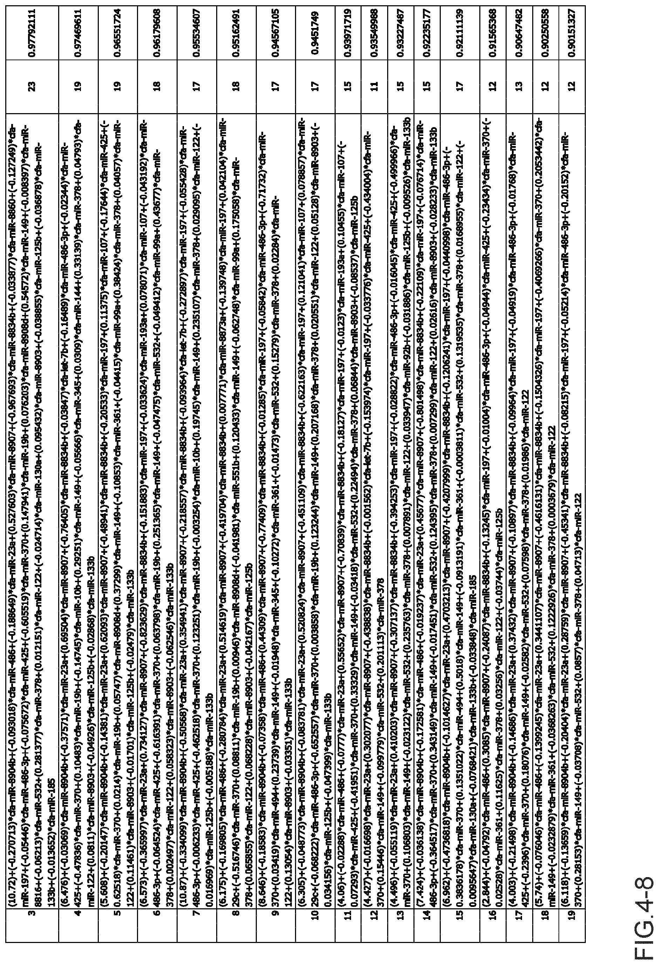

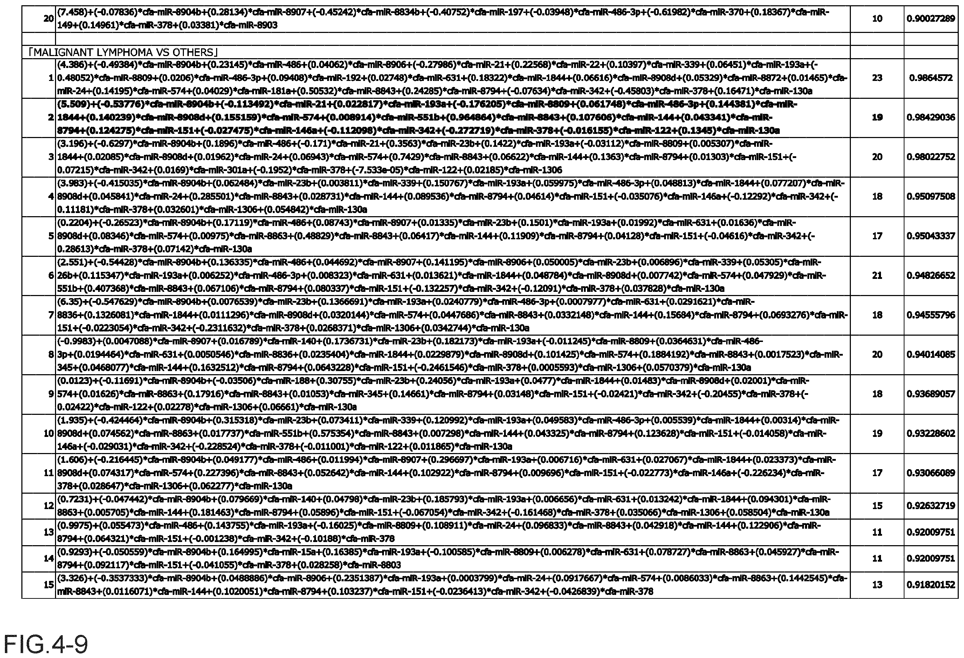

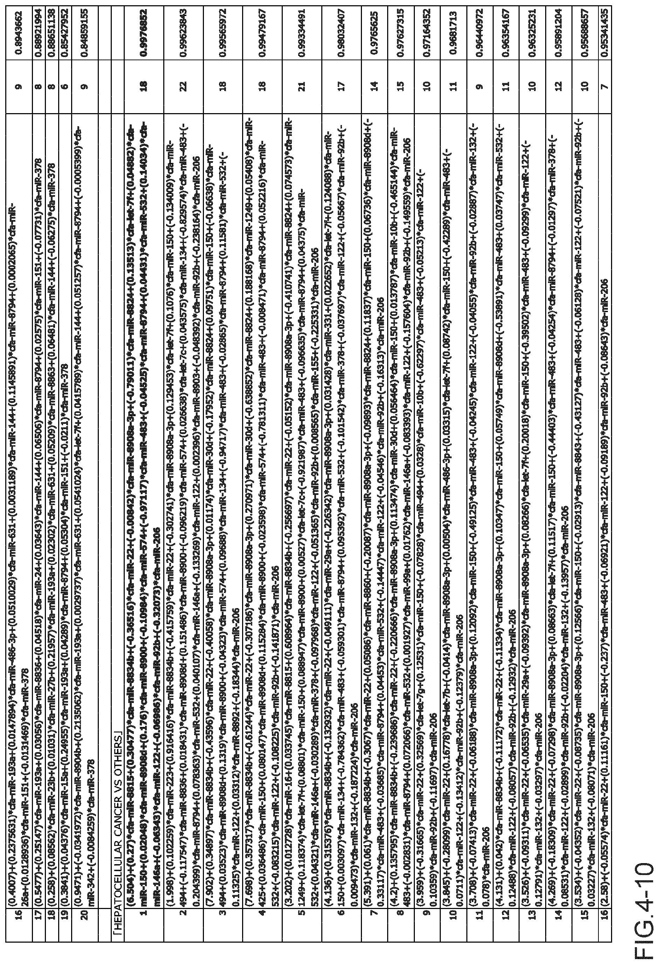

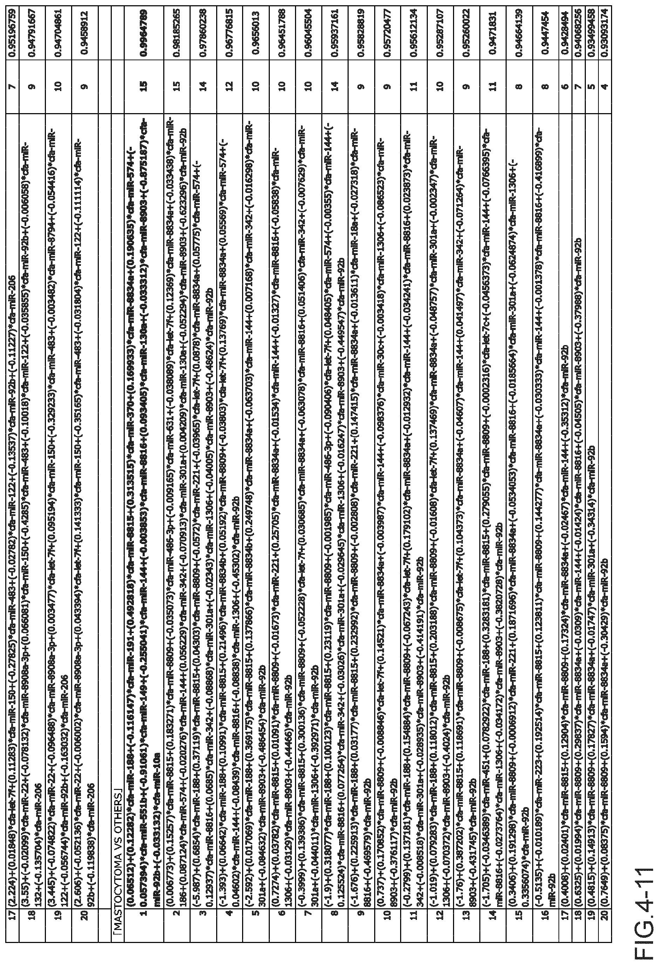

[FIG. 4-5] Figures showing discriminants sampled in the implementation of the present invention and the AUCs thereof.
[FIG. 4-6] Figures showing discriminants sampled in the implementation of the present invention and the AUCs thereof.
[FIG. 4-7] Figures showing discriminants sampled in the implementation of the present invention and the AUCs thereof.
[FIG. 4-8] Figures showing discriminants sampled in the implementation of the present invention and the AUCs thereof.
[FIG. 4-9] Figures showing discriminants sampled in the implementation of the present invention and the AUCs thereof.
[FIG. 4-10] Figures showing discriminants sampled in the implementation of the present invention and the AUCs thereof.
[FIG. 4-11] Figures showing discriminants sampled in the implementation of the present invention and the AUCs thereof.

### Description of Embodiments

### [Steps included in method for diagnosing canine cancer]

Although a method for diagnosing canine cancer of the present invention will be divided into some steps and specifically described here, the modifications such as the order, the separation, and the integration of the steps included in the method are possible as long as the modifications does not depart from an object of the invention.

### <Step 1: Expression analysis of microRNA>

Dog body fluid is collected as a sample (specimen), a microRNA is extracted from the collected body fluid, and the gene expression of the extracted microRNA is analyzed, and the expression level of the microRNA is detected.

As the sample, body fluid of a dog suffering from cancer including a "specific cancer disease" is provided besides body fluid of a healthy dog. Within the scope of the present disclosure, the "specific cancer disease" may mean a cancer disease to be diagnosed.

Examples of the body fluid include serum, saliva, or urine. Dog serum is preferably used.

Although the "specific cancer disease" can be selected from the group consisting of intraoral melanoma, urothelial cancer, malignant lymphoma, hepatocellular cancer, and mastocytoma, which occur relatively frequently as canine cancer, the "specific cancer disease" is not limited to these.

As a technique for gene expression analysis, for example, a microarray analysis, next-generation sequencing (NGS), or the like can be used. As long as the gene expression of the microRNA extracted from dog body fluid can be detected, another technique well-known in the art may be adopted. Since, in the gene expression analysis to be performed here, the interrelationship between cancer diseases and the gene expression levels needs to be extensively analyzed, it is however desirable to adopt a technique that enables analyzing a microRNA extracted from dog body fluid as comprehensively as possible. Microarrays analysis is preferably usable due to this. Since techniques for gene expression analysis including a microarray analysis are known, detailed description is omitted here. Since the results of the gene expression analysis reaches an enormous amount of data, it is desirable to make a computer execute the gene expression analysis including operations for putting the genes expression level of the microRNA in order.

### <Step 2: Sampling of specific microRNA>

MicroRNA showing a significant difference in the gene expression level between a dog suffering from a "specific cancer disease" and a healthy dog, or, in a specific embodiment, showing a significant difference in the gene expression level among a dog suffering from a specific cancer disease, a dog suffering from another cancer disease, and a healthy dog, is sampled based on the results of the expression analysis of the microRNA. The number of the sampled microRNAs depends on the sampling technique and objects thereof here. Although the number is not limited to the following number, the number is commonly around 1 to 80, and preferably around 4 to 50.

Within the present disclosure, such microRNA is referred to a "specific microRNA".

Although the "specific cancer disease" can be selected from the group consisting of intraoral melanoma, urothelial cancer, malignant lymphoma, hepatocellular cancer, and mastocytoma, which occur relatively frequently as canine cancer, the "specific cancer disease" is not limited to these.

As the method for sampling the "specific microRNA", a technique involving performing the statistical analysis processing based on the results of the gene expression analysis of the microRNA, making a model discriminant for predicting a canine cancer, and sampling a "specific microRNA" from such a discriminant can be adopted.

Examples of the specific technique for statistical analysis processing include LASSO (at least absolute shrinkage and selection operator) regression analysis. As long as the object of the invention can be achieved, the technique is not limited to this.

In a specific embodiment, a technique involving performing LASSO regression analysis based on the results of the gene expression analysis of microRNA, making a model discriminant for predicting a canine cancer, and sampling a "specific microRNA" from such a discriminant can be adopted as the method for sampling the "specific microRNA".

The LASSO regression analysis is known as a regression analysis technique for improving the predictive accuracy and the interpretability of the statistical model to be generated, and a technique using the LASSO regression analysis is particularly preferable for sampling more characteristic and/or frequent microRNA in the "specific cancer disease" as the "specific microRNA" available as a biomarker in the subsequent diagnostic step.

Examples of other techniques for sampling a "specific microRNA" include a technique involving generating a heat map image based on the results of the gene expression analysis, mechanically selecting microRNAs showing a marked difference in the gene expression level of between a dog suffering from a "specific cancer disease" and a healthy dog (including a dog suffering from another cancer disease in some cases), for example, a two or more-fold difference, by image analysis processing with a computer, and sampling a "specific microRNA" from such microRNAs. The heat map image is an image in which the genetic expression levels are visualized, and shows whether the genetic expression levels are high or low with the types and the shades of the used colors.

As another technique for sampling a "specific microRNA", a technique involving generating a heat map image that enables recognizing the results of the expression analysis of such microRNAs intuitively and further performing clustering processing and the like, followed by the combination with a technique using the LASSO regression analysis or a technique involving generating a heat map image as the preprocessing of the LASSO regression analysis may accordingly be adopted.

Furthermore, a technique for sampling a "specific microRNA" based by the image analysis processing based on the machine learning and the like using AI is also assumed as the technique for sampling a "specific microRNA", this is not excluded in the present invention. It is expected that such image analysis processing by AI enables sampling microRNA difficultly sampled by simple threshold setting in the case where a significant difference is shown only by combining multiple microRNAs, the case where even though the difference is slight, a significant difference is shown, and the like.

It is desirable to make a computer execute the sampling of the "specific microRNA" also in the case where a heat map image that requires mapping the gene expression level accurately is generated. Such a computer may be a computer in which software separately developed for implementing the present invention is installed, or may be a computer in which a commercial software developed for the gene expression analysis, for example, a microarray analysis is installed.

### <Step 3: Diagnosis with specific microRNA>

The result of the gene expression analysis of the microRNA extracted from the body fluid of the dog to be diagnosed is acquired newly, the gene expression level of the extracted "specific microRNA" is determined using the result, and the degree of a risk of the dog to be diagnosed being suffering from the "specific cancer disease" is diagnosed using the gene expression level as a diagnostic criterion.

The technique to be used for the expression analysis of the microRNA extracted from the body fluid of the dog to be diagnosed may be the same as or different from the technique for the gene expression analysis in the step 1. For example, the microarray analysis can be adopted, but the technique is not limited to this.

Although examples of the body fluid include serum, saliva, or urine, dog serum is preferably used.

The degree of the risk may be an aspect showing that the dog is diagnosed as "having the cancer" or "not having the cancer" alternatively, or may be an aspect showing that the dog is diagnosed as "having a XX% chance of having the cancer" in multiple steps. In a specific embodiment, the degree of the risk may include an aspect showing diagnosis like "the risk of being suffering from the specific cancer is higher/lower than the risk of being suffering from another cancer disease".

Since the "specific microRNA" that is supposed to be particularly highly relevant to the specific cancer disease is sampled beforehand, according to the present invention, the subject to be diagnosed only has to be analyzed for the expression of commonly around 1 to 80 "specific microRNAs", preferably around 4 to 50 "specific microRNAs", and the expression does not need to be extensively and comprehensively analyzed. In short, the microRNA of the dog to be diagnosed can be efficiently subjected to gene expression analysis, so that the processing burden to be imposed on the diagnosis thereof is reduced, and the dog can be rapidly and accurately diagnosed.

Accordingly, it is desirable that the diagnosis be executed with a computer, and the diagnosis can also be executed with a personal computer, a smartphone, or the like used at standard home due to the relatively light processing burden thereon.

### [Biomarker to be used for diagnosing canine cancer]

Hereinafter, the "specific microRNA" sampled in the implementation of the present invention will be specifically shown for each diagnostic purpose/use for which the utilization thereof as the biomarker is assumed.

### <microRNAs to be used for diagnosing five cancer diseases>

As the microRNAs that is effective for distinguishing a dog suffering from any of five cancer diseases including intraoral melanoma, urothelial cancer, malignant lymphoma, hepatocellular cancer, and mastocytoma from a healthy dog, the following 44 microRNAs were sampled:
cfa-let-7c, cfa-miR-103, cfa-miR-10a, cfa-miR-122, cfa-miR-125a, cfa-miR-125b, cfa-miR-126, cfa-miR-130a, cfa-miR-144, cfa-miR-150, cfa-miR-155, cfa-miR-186, cfa-miR-193a, cfa-miR-197, cfa-miR-199, cfa-miR-19b, cfa-miR-22, cfa-miR-222, cfa-miR-223, cfa-miR-24, cfa-miR-26a, cfa-miR-27b, cfa-miR-339, cfa-miR-342, cfa-miR-378, cfa-miR-383, cfa-miR-483, cfa-miR-486-3p, cfa-miR-489, cfa-miR-551b, cfa-miR-660, cfa-miR-718, cfa-miR-874, cfa-miR-8794, cfa-miR-8798, cfa-miR-8843, cfa-miR-8859a, cfa-miR-8860, cfa-miR-8903, cfa-miR-8906, cfa-miR-8907, cfa-miR-8908a-3p, cfa-miR-8908d and cfa-miR-92b.

The frequencies and the nucleotide sequences of the microRNAs are shown below.

**[Table 1]**

| miRNA | Frequency | Accession No. | Seq |
|---|---|---|---|
| cfa-let-7c | 1 | MIMAT0006669 | ugagguaguagguuguaugguu |
| cfa-miR-103 | 2 | MIMAT0006687 | agcagcauuguacagggcuauga |
| cfa-miR-10a | 3 | MIMAT0006737 | uacccuguagauccgaauuugu |
| cfa-miR-122 | 18 | MIMAT0006619 | uggagugugacaaugguguuug |
| cfa-miR-125a | 20 | MIMAT0006609 | ucccugagacccuuuaaccugu |
| cfa-miR-125b | 1 | MIMAT0006670 | ucccugagacccuaacuuguga |
| cfa-miR-126 | 20 | MIMAT0006730 | cauuauuacuuuugguacgcg |
| cfa-miR-130a | 1 | MIMAT0006631 | cagugcaauguuaaaagggcau |
| cfa-miR-144 | 20 | MIMAT0006734 | uacaguauagaugauguacuag |
| cfa-miR-150 | 20 | MIMAT0006602 | ucucccaacccuuguaccagug |
| cfa-miR-155 | 15 | MIMAT0006671 | uuaaugcuaaucgugauaggggu |
| cfa-miR-186 | 18 | MIMAT0006694 | caaagaauucuccuuuugggcu |
| cfa-miR-193a | 12 | MIMAT0006735 | ugggucuuugcgggcgagauga |
| cfa-miR-197 | 20 | MIMAT0006698 | uucaccaccuucuccacccagc |
| cfa-miR-199 | 15 | MIMAT0006642 | acaguagucugcacauugguu |
| cfa-miR-19b | 6 | MIMAT0006652 | ugugcaaauccaugcaaaacug |
| cfa-m i R-22 | 2 | MIMAT0006733 | aagcugccaguugaagaacugu |
| cfa-miR-222 | 1 | MIMAT0009851 | agcuacaucuggcuacugggu |
| cfa-miR-223 | 1 | MIMAT0009852 | ugucaguuugucaaauacccc |
| cfa-miR-24 | 20 | MIMAT0006614 | uggcucaguucagcaggaacagg |
| cfa-miR-26a | 2 | MIMAT0006595 | uucaaguaauccaggauaggcu |
| cfa-miR-27b | 1 | MIMAT0006613 | uucacaguggcuaaguucugc |
| cfa-miR-339 | 3 | MIMAT0011134 | ucccuguccuccaggagcu |
| cfa-miR-342 | 1 | MIMAT0006709 | ucucacacagaaaucgcacccgu |
| cfa-miR-378 | 10 | MIMAT0006683 | acuggacuuggagucagaaggc |
| cfa-miR-383 | 9 | MIMAT0006629 | agaucagaaggugauuguggcu |
| cfa-miR-483 | 20 | MIMAT0009901 | ucacuccuccccucccgucuu |
| cfa-miR-486-3p | 2 | MIMAT0032036 | ucggggcagcucaguacaggau |
| cfa-miR-489 | 3 | MIMAT0009860 | gugacaucacauauacggcggc |
| cfa-miR-551b | 1 | MIMAT0009913 | gcgacccauacuugguuucag |
| cfa-miR-660 | 3 | MIMAT0006760 | uacccauugcauaucggaguug |
| cfa-miR-718 | 3 | MIMAT0009939 | cuuccgccccgccgggcgccg |
| cfa-miR-874 | 2 | MIMAT0009930 | cugcccuggcccgagggaccga |
| cfa-miR-8794 | 1 | MIMAT0034284 | ugccccaucaucagccuccccagu |
| cfa-miR-8798 | 19 | MIMAT0034288 | ugcggucgaugcgaggccccgg |
| cfa-miR-8843 | 12 | MIMAT0034336 | uuguuuuuuucucucgccccgccug |
| cfa-miR-8859a | 3 | MIMAT0034354 | uggaucggagccgggguccgga |
| cfa-miR-8860 | 2 | MIMAT0034355 | uacacuagguuuggaggaaagugg g |
| cfa-miR-8903 | 3 | MIMAT0034414 | ucuugggccccacccccggagacu |
| cfa-miR-8906 | 18 | MIMAT0034434 | uccucugcauuuggcugggacggca |
| cfa-miR-8907 | 20 | MIMAT0034435 | ugccgauucugaagugggaaga |
| cfa-miR-8908a-3p | 16 | MIMAT0034429 | uaauuaggaccucccugagcggagu |
| cfa-miR-8908d | 3 | MIMAT0034420 | auuagcgccugacugagugggguc |
| cfa-miR-92b | 20 | MIMAT0006703 | uauugcacucgucccggccucc |

The microRNAs are microRNAs included in the top 20 discriminants selected as discriminants including characteristic and/or frequent microRNAs in the five cancer diseases after the model discriminants for predicting a specific cancer disease are made by statistical analysis processing using the LASSO regression analysis based on the results of the gene expression analysis of microRNAs extracted from the dog suffering from any of the five cancer diseases including intraoral melanoma, urothelial cancer, malignant lymphoma, hepatocellular cancer, and mastocytoma and the healthy dog. The discriminants and the AUCs indicating the discrimination abilities thereof are shown in FIG. 4 ("healthy vs all cancers (five types)").

In the implementation of the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, or all the 44 of the microRNAs are determined, and the degree of the risk of the dog to be diagnosed being suffering from any of intraoral melanoma, urothelial cancer, malignant lymphoma, hepatocellular cancer, and mastocytoma can be diagnosed based on the gene expression levels.

Both of microRNA at a particularly high gene expression level and microRNA at a particularly low gene expression level can be included in the "specific microRNA" as compared with the healthy dog in five cancer diseases including intraoral melanoma, urothelial cancer, malignant lymphoma, hepatocellular cancer, and mastocytoma.

Accordingly, it is desirable in the diagnosis to diagnose a dog having a high gene expression level of at least one microRNA selected from the group consisting of cfa-miR-483, cfa-miR-718, cfa-miR-8794, cfa-miR-8798, cfa-miR-8859a, cfa-miR-8908a-3p, and cfa-miR-92b as being at a high risk of being suffering from any of intraoral melanoma, urothelial cancer, malignant lymphoma, hepatocellular cancer, and mastocytoma and/or to diagnose a dog having a high gene expression level of at least one microRNA selected from the group consisting of cfa-let-7c, cfa-miR-103, cfa-miR-10a, cfa-miR-125a, cfa-miR-125b, cfa-miR-126, cfa-miR-144, cfa-miR-150, cfa-miR-155, cfa-miR-186, cfa-miR-199, cfa-miR-19b, cfa-miR-24, cfa-miR-26a, cfa-miR-383, cfa-miR-489, cfa-miR-8907, and cfa-miR-8908d as being at a low risk of being suffering from any of intraoral melanoma, urothelial cancer, malignant lymphoma, hepatocellular cancer, and mastocytoma.

It is believed that the diagnosis of canine cancer using the "specific microRNA" as a biomarker is useful for inclusively examining whether a dog does not suffer from relatively frequent specific cancer disease in cancer screening or the like, and is the most highly required.

### <microRNA to be used for the diagnosing intraoral melanoma>

Examples of a biomarker that is effective for distinguishing the diagnosis of a dog suffering from intraoral melanoma from a healthy dog include 22 microRNAs shown below:
cfa-miR-10a, cfa-miR-1185, cfa-miR-125a, cfa-miR-126, cfa-miR-144, cfa-miR-146a, cfa-miR-149, cfa-miR-150, cfa-miR-155, cfa-miR-184, cfa-miR-186, cfa-miR-197, cfa-miR-199, cfa-miR-19b, cfa-miR-24, cfa-miR-30c, cfa-miR-483, cfa-miR-489, cfa-miR-8798, cfa-miR-8816, cfa-miR-8875, and cfa-miR-8908a-3p.

The frequencies and the nucleotide sequences of the microRNAs are shown below.

**[Table 2]**

| miRNA | Frequenc y | Accession No. | Seq |
|---|---|---|---|
| cfa-miR-10a | 4 | MIMAT0006737 | uacccuguagauccgaauuugu |
| cfa-miR-1185 | 2 | MIMAT0034383 | auauacagggggagacucuuau |
| cfa-miR-125a | 20 | MIMAT0006609 | ucccugagacccuuuaaccugu |
| cfa-miR-126 | 20 | MIMAT0006730 | cauuauuacuuuugguacgcg |
| cfa-miR-144 | 20 | MIMAT0006734 | uacaguauagaugauguacuag |
| cfa-miR-146a | 2 | MIMAT0006684 | ugagaacugaauuccauggguu |
| cfa-miR-149 | 1 | MIMAT0009884 | ucuggcuccgugucuucacuccc |
| cfa-miR-150 | 2 | MIMAT0006602 | ucucccaacccuuguaccagug |
| cfa-miR-155 | 4 | MIMAT0006671 | uuaaugcuaaucgugauaggggu |
| cfa-miR-184 | 18 | MIMAT0009842 | uggacggagaacugauaagggu |
| cfa-miR-186 | 2 | MIMAT0006694 | caaagaauucuccuuuugggcu |
| cfa-miR-197 | 20 | MIMAT0006698 | uucaccaccuucuccacccagc |
| cfa-miR-199 | 16 | MIMAT0006642 | acaguagucugcacauugguu |
| cfa-miR-19b | 1 | MIMAT0006652 | ugugcaaauccaugcaaaacug |
| cfa-miR-24 | 14 | MIMAT0006614 | uggcucaguucagcaggaacagg |
| cfa-miR-30c | 1 | MIMAT0006605 | uguaaacauccuacacucucagcu |
| cfa-miR-483 | 1 | MIMAT0009901 | ucacuccuccccucccgucuu |
| cfa-miR-489 | 1 | MIMAT0009860 | gugacaucacauauacggcggc |
| cfa-miR-8798 | 18 | MIMAT0034288 | ugcggucgaugcgaggccccgg |
| cfa-miR-8816 | 4 | MIMAT0034306 | uagaauccaggucaugugacuccc |
| cfa-miR-8875 | 1 | MIMAT0034375 | ugcuguagcggaacccggggcggg c |
| cfa-miR-8908a-3p | 1 | MIMAT0034429 | uaauuaggaccucccugagcggag u |

The microRNAs are microRNAs included in the top 20 discriminants selected as discriminants including characteristic and/or frequent microRNAs in intraoral melanoma after model discriminants for predicting intraoral melanoma are made by statistical analysis processing using the LASSO regression analysis based on the results of the gene expression analysis of the microRNAs extracted from the dog suffering from intraoral melanoma and the healthy dog. The discriminants and the AUCs indicating the discrimination abilities thereof are shown in FIG. 4 ("healthy vs intraoral melanoma").

According to the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, and all the 22 of the microRNAs are determined, and the degree of the risk of the dog being suffering from intraoral melanoma can be diagnosed based on the gene expression levels.

Both of microRNA at a particularly high gene expression level and microRNA at a particularly low gene expression level can be included in the "specific microRNA" as compared with the healthy dog in intraoral melanoma.

Accordingly, it is desirable in the diagnosis to diagnose a dog having a high gene expression level of at least one microRNA selected from the group consisting of cfa-miR-197, cfa-miR-483, cfa-miR-8798, cfa-miR-8875, and cfa-miR-8908a-3p as being at a high risk of being suffering from intraoral melanoma, and/or to diagnose a dog having a high gene expression level of at least one microRNA selected from the group consisting of cfa-miR-10a, cfa-miR-125a, cfa-miR-126, cfa-miR-144, cfa-miR-146a, cfa-miR-155, cfa-miR-184, cfa-miR-186, cfa-miR-199, cfa-miR-19b, cfa-miR-24, cfa-miR-30c, and cfa-miR-489 as being at a low risk of being suffering from intraoral melanoma.

It is believed that the diagnosis of canine cancer using the "specific microRNA" as a biomarker is required for the monitoring of the recurrence of intraoral melanoma after the treatment thereof, and the like besides cancer screening or the like.

### <microRNA to be used for diagnosing urothelial cancer>

Examples of a biomarker that is effective for distinguishing the diagnosis of a dog suffering from urothelial cancer from a healthy dog include 41 microRNAs shown below:
cfa-let-7f, cfa-miR-10a, cfa-miR-1249, cfa-miR-125a, cfa-miR-126, cfa-miR-1306, cfa-miR-130a, cfa-miR-133b, cfa-miR-144, cfa-miR-146a, cfa-miR-149, cfa-miR-150, cfa-miR-155, cfa-miR-1844, cfa-miR-186, cfa-miR-193a, cfa-miR-197, cfa-miR-19b, cfa-miR-2114, cfa-miR-24, cfa-miR-29b, cfa-miR-339, cfa-miR-342, cfa-miR-361, cfa-miR-370, cfa-miR-425, cfa-miR-483, cfa-miR-494, cfa-miR-502, cfa-miR-551b, cfa-miR-660, cfa-miR-665, cfa-miR-718, cfa-miR-8798, cfa-miR-8824, cfa-miR-8832, cfa-miR-8834a, cfa-miR-8843, cfa-miR-8904b, cfa-miR-8907 and cfa-miR-8908a-3p. The frequencies and the nucleotide sequences of the microRNAs are shown below.

**[Table 3]**

| miRNA | Frequency | Accession No. | Seq |
|---|---|---|---|
| cfa-let-7f | 1 | MIMAT0006610 | ugagguaguagauuguauaguu |
| cfa-miR-10a | 1 | MIMAT0006737 | uacccuguagauccgaauuugu |
| cfa-miR-1249 | 1 | MIMAT0034321 | acgcccuucccccccuucuuca |
| cfa-miR-125a | 15 | MIMAT0006609 | ucccugagacccuuuaaccugu |
| cfa-miR-126 | 12 | MIMAT0006730 | cauuauuacuuuugguacgcg |
| cfa-miR-1306 | 4 | MIMAT0006661 | ccaccuccccugcaaacgucc |
| cfa-miR-130a | 1 | MIMAT0006631 | cagugcaauguuaaaagggcau |
| cfa-miR-133b | 1 | MIMAT0009835 | uuugguccccuucaaccagcua |
| cfa-miR-144 | 20 | MIMAT0006734 | uacaguauagaugauguacuag |
| cfa-miR-146a | 1 | MIMAT0006684 | ugagaacugaauuccauggguu |
| cfa-miR-149 | 1 | MIMAT0009884 | ucuggcuccgugucuucacuccc |
| cfa-miR-150 | 3 | MIMAT0006602 | ucucccaacccuuguaccagug |
| cfa-miR-155 | 4 | MIMAT0006671 | uuaaugcuaaucgugauaggggu |
| cfa-miR-1844 | 9 | MIMAT0006740 | aggacuacggacgggcugag |
| cfa-miR-186 | 19 | MIMAT0006694 | caaagaauucuccuuuugggcu |
| cfa-miR-193a | 6 | MIMAT0006735 | ugggucuuugcgggcgagauga |
| cfa-miR-197 | 20 | MIMAT0006698 | uucaccaccuucuccacccagc |
| cfa-miR-19b | 2 | MIMAT0006652 | ugugcaaauccaugcaaaacug |
| cfa-miR-2114 | 2 | MIMAT0034427 | uagucccuuccuugaaggaucggc |
| cfa-miR-24 | 20 | MIMAT0006614 | uggcucaguucagcaggaacagg |
| cfa-miR-29b | 4 | MIMAT0006625 | uagcaccauuugaaaucaguguu |
| cfa-miR-339 | 2 | MIMAT0011134 | ucccuguccuccaggagcu |
| cfa-miR-342 | 1 | MIMAT0006709 | ucucacacagaaaucgcacccgu |
| cfa-miR-361 | 3 | MIMAT0006751 | uuaucagaaucuccagggguac |
| cfa-miR-370 | 17 | MIMAT0009889 | gccugcugggguggaaccuggu |
| cfa-miR-425 | 16 | MIMAT0006639 | aaugacacgaucacucccguuga |
| cfa-miR-483 | 8 | MIMAT0009901 | ucacuccuccccucccgucuu |
| cfa-miR-494 | 5 | MIMAT0009905 | ugaaacauacacgggaaaccuc |
| cfa-miR-502 | 1 | MIMAT0006761 | aaugcaccugggcaaggauuca |
| cfa-miR-551b | 2 | MIMAT0009913 | gcgacccauacuugguuucag |
| cfa-miR-660 | 3 | MIMAT0006760 | uacccauugcauaucggaguug |
| cfa-miR-665 | 15 | MIMAT0009934 | accaggaggcuaaggccccu |
| cfa-miR-718 | 2 | MIMAT0009939 | cuuccgccccgccgggcgccg |
| cfa-miR-8798 | 1 | MIMAT0034288 | ugcggucgaugcgaggccccgg |
| cfa-miR-8824 | 1 | MIMAT0034314 | guuuccaucuccacccccggca |
| cfa-miR-8832 | 1 | MIMAT0034325 | uccagggguaggauugauuguggga |
| cfa-miR-8834a | 1 | MIMAT0034327 | ugccgggccuggaggcuccgggg |
| cfa-miR-8843 | 4 | MIMAT0034336 | uuguuuuuuucucucgccccgccug |
| cfa-miR-8904b | 1 | MIMAT0034424 | uaacagcaccugcgccccggggaga |
| cfa-miR-8907 | 20 | MIMAT0034435 | ugccgauucugaagugggaaga |
| cfa-miR-8908a-3p | 3 | MIMAT0034429 | uaauuaggaccucccugagcggagu |

The microRNAs are microRNAs included in the top 20 discriminants selected as discriminants including characteristic and/or frequent microRNAs in urothelial cancer after model discriminants for predicting urothelial cancer are made by statistical analysis processing using the LASSO regression analysis based on the results of the gene expression analysis of the microRNAs extracted from the dog suffering from urothelial cancer and the healthy dog. The discriminants and the AUCs indicating the discrimination abilities thereof are shown in FIG. 4 ("healthy vs urothelial cancer ").

According to the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or all the 41 of the microRNAs are determined, and the degree of the risk of the dog being suffering from urothelial cancer can be diagnosed based on the gene expression levels.

Both of microRNA at a particularly high gene expression level and microRNA at a particularly low gene expression level can be included in the "specific microRNA" as compared with the healthy dog in urothelial cancer.

Accordingly, it is desirable in the diagnosis to diagnose a dog having a high gene expression level of at least one microRNA selected from the group consisting of cfa-miR-483, cfa-miR-718, and cfa-miR-8908a-3p as being at a high risk of being suffering from urothelial cancer, and/or to diagnose a dog having a high gene expression level of at least one microRNA selected from the group consisting of cfa-miR-125a, cfa-miR-126, cfa-miR-144, cfa-miR-146a, cfa-miR-149, cfa-miR-150, cfa-miR-155, cfa-miR-186, cfa-miR-2114, cfa-miR-24, cfa-miR-29b, cfa-miR-8832, and cfa-miR-8907 as being at a low risk of being suffering from urothelial cancer.

It is believed that the diagnosis of canine cancer using the "specific microRNA" as a biomarker is required for the monitoring of the recurrence of urothelial cancer after the treatment thereof, and the like besides cancer screening or the like.

### <microRNA to be used for diagnosing malignant lymphoma>

Examples of a biomarker that is effective for distinguishing a dog suffering from malignant lymphoma from a healthy dog include 24 microRNAs shown below:
cfa-miR-1185, cfa-miR-122, cfa-miR-1249, cfa-miR-126, cfa-miR-1306, cfa-miR-144, cfa-miR-150, cfa-miR-184, cfa-miR-188, cfa-miR-197, cfa-miR-199, cfa-miR-24, cfa-miR-342, cfa-miR-345, cfa-miR-378, cfa-miR-425, cfa-miR-574, cfa-miR-8794, cfa-miR-8797, cfa-miR-8798, cfa-miR-8900, cfa-miR-8904b, cfa-miR-8907 and cfa-miR-8908d.

The frequencies and the nucleotide sequences of the microRNAs is shown below.

**[Table 4]**

| miRNA | Frequency | Accession No. | Seq |
|---|---|---|---|
| cfa-miR-1185 | 4 | MIMAT0034383 | auauacagggggagacucuuau |
| cfa-miR-122 | 8 | MIMAT0006619 | uggagugugacaaugguguuug |
| cfa-miR-1249 | 7 | MIMAT0034321 | acgcccuucccccccuucuuca |
| cfa-miR-126 | 15 | MIMAT0006730 | cauuauuacuuuugguacgcg |
| cfa-miR-1306 | 2 | MIMAT0006661 | ccaccuccccugcaaacgucc |
| cfa-miR-144 | 20 | MIMAT0006734 | uacaguauagaugauguacuag |
| cfa-miR-150 | 2 | MIMAT0006602 | ucucccaacccuuguaccagug |
| cfa-miR-184 | 1 | MIMAT0009842 | uggacggagaacugauaagggu |
| cfa-miR-188 | 1 | MIMAT0009880 | caucccuugcaugguggagggu |
| cfa-miR-197 | 6 | MIMAT0006698 | uucaccaccuucuccacccagc |
| cfa-miR-199 | 18 | MIMAT0006642 | acaguagucugcacauugguu |
| cfa-miR-24 | 17 | MIMAT0006614 | uggcucaguucagcaggaacagg |
| cfa-miR-342 | 1 | MIMAT0006709 | ucucacacagaaaucgcacccgu |
| cfa-miR-345 | 2 | MIMAT0006710 | ccugaacuaggggucuggagg |
| cfa-miR-378 | 17 | MIMAT0006683 | acuggacuuggagucagaaggc |
| cfa-miR-425 | 2 | MIMAT0006639 | aaugacacgaucacucccguuga |
| cfa-miR-574 | 2 | MIMAT0006673 | cacgcucaugcacacacccaca |
| cfa-miR-8794 | 3 | MIMAT0034284 | ugccccaucaucagccuccccagu |
| cfa-miR-8797 | 11 | MIMAT0034287 | uugccaaggacugagagcucggg |
| cfa-miR-8798 | 9 | MIMAT0034288 | ugcggucgaugcgaggccccgg |
| cfa-miR-8900 | 5 | MIMAT0034410 | uaggacuuuaauggcuggagaga |
| cfa-miR-8904b | 5 | MIMAT0034424 | uaacagcaccugcgccccggggaga |
| cfa-miR-8907 | 20 | MIMAT0034435 | ugccgauucugaagugggaaga |
| cfa-miR-8908d | 8 | MIMAT0034420 | auuagcgccugacugagugggguc |

The microRNAs are microRNAs included in the top 20 discriminants selected as discriminants including characteristic and/or frequent microRNAs in malignant lymphoma after model discriminants for predicting malignant lymphoma are made by statistical analysis processing using the LASSO regression analysis based on the results of the gene expression analysis of the microRNAs extracted from the dog suffering from malignant lymphoma and the healthy dog. The discriminants and the AUCs indicating the discrimination abilities thereof are shown in FIG. 4 ("healthy vs malignant lymphoma").

According to the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, 22, 23, all the 24 of the microRNAs are determined, and the degree of the risk of the dog being suffering from malignant lymphoma can be diagnosed based on the gene expression levels.

Both of microRNA at a particularly high gene expression level and microRNA at a particularly low gene expression level can be included in the "specific microRNA" as compared with the healthy dog in malignant lymphoma.

Accordingly, it is desirable in the diagnosis to diagnose a dog having a high gene expression level of at least one microRNA selected from the group consisting of cfa-miR-188, cfa-miR-342, cfa-miR-378, cfa-miR-8797, cfa-miR-8798, and cfa-miR-8904b as being at a high risk of being suffering from malignant lymphoma, and/or to diagnose a dog having a high gene expression level of at least one microRNA selected from the group consisting of cfa-miR-126, cfa-miR-1306, cfa-miR-144, cfa-miR-184, cfa-miR-199, cfa-miR-24, cfa-miR-345, cfa-miR-574, cfa-miR-8900, cfa-miR-8907, and cfa-miR-8908d as being at a low risk of being suffering from malignant lymphoma.

It is believed that the diagnosis of canine cancer using the "specific microRNA" as a biomarker is required for the monitoring of the recurrence of malignant lymphoma after the treatment thereof, and the like besides cancer screening or the like.

### <microRNA to be used for diagnosing hepatocellular cancer>

Examples of a biomarker that is effective for distinguishing a dog suffering from hepatocellular cancer from a healthy dog include 28 microRNAs shown below:
cfa-let-7g, cfa-miR-10a, cfa-miR-1185, cfa-miR-122, cfa-miR-125b, cfa-miR-150, cfa-miR-155, cfa-miR-197, cfa-miR-199, cfa-miR-206, cfa-miR-22, cfa-miR-378, cfa-miR-383, cfa-miR-483, cfa-miR-665, cfa-miR-7, cfa-miR-718, cfa-miR-874, cfa-miR-8794, cfa-miR-8798, cfa-miR-8875, cfa-miR-8900, cfa-miR-8902, cfa-miR-8907, cfa-miR-8908a-3p, cfa-miR-8908d, cfa-miR-92b and cfa-miR-99a.

The frequencies and the nucleotide sequences of the microRNAs is shown below.

**[Table 5]**

| miRNA | Frequenc y | Accession No. | Seq |
|---|---|---|---|
| cfa-let-7g | 5 | MIMAT0006637 | ugagguaguaguuuguacaguu |
| cfa-miR-10a | 19 | MIMAT0006737 | uacccuguagauccgaauuugu |
| cfa-miR-1185 | 5 | MIMAT0034383 | auauacagggggagacucuuau |
| cfa-miR-122 | 19 | MIMAT0006619 | uggagugugacaaugguguuug |
| cfa-miR-125b | 2 | MIMAT0006670 | ucccugagacccuaacuuguga |
| cfa-miR-150 | 19 | MIMAT0006602 | ucucccaacccuuguaccagug |
| cfa-miR-155 | 1 | MIMAT0006671 | uuaaugcuaaucgugauaggggu |
| cfa-miR-197 | 4 | MIMAT0006698 | uucaccaccuucuccacccagc |
| cfa-miR-199 | 8 | MIMAT0006642 | acaguagucugcacauugguu |
| cfa-miR-206 | 17 | MIMAT0006606 | uggaauguaaggaagugugugg |
| cfa-miR-22 | 9 | MIMAT0006733 | aagcugccaguugaagaacugu |
| cfa-miR-378 | 13 | MIMAT0006683 | acuggacuuggagucagaaggc |
| cfa-miR-383 | 7 | MIMAT0006629 | agaucagaaggugauuguggcu |
| cfa-miR-483 | 11 | MIMAT0009901 | ucacuccuccccucccgucuu |
| cfa-miR-665 | 18 | MIMAT0009934 | accaggaggcuaaggccccu |
| cfa-miR-7 | 9 | MIMAT0006634 | uggaagacuagugauuuuguugu |
| cfa-miR-718 | 13 | MIMAT0009939 | cuuccgccccgccgggcgccg |
| cfa-miR-874 | 5 | MIMAT0009930 | cugcccuggcccgagggaccga |
| cfa-miR-8794 | 16 | MIMAT0034284 | ugccccaucaucagccuccccagu |
| cfa-miR-8798 | 5 | MIMAT0034288 | ugcggucgaugcgaggccccgg |
| cfa-miR-8875 | 11 | MIMAT0034375 | ugcuguagcggaacccggggcgggc |
| cfa-miR-8900 | 19 | MIMAT0034410 | uaggacuuuaauggcuggagaga |
| cfa-miR-8902 | 12 | MIMAT0034412 | cauccauuucuuucaccuggggaaa |
| cfa-miR-8907 | 20 | MIMAT0034435 | ugccgauucugaagugggaaga |
| cfa-miR-8908a-3p | 20 | MIMAT0034429 | uaauuaggaccucccugagcggagu |
| cfa-miR-8908d | 1 | MIMAT0034420 | auuagcgccugacugagugggguc |
| cfa-miR-92b | 20 | MIMAT0006703 | uauugcacucgucccggccucc |
| cfa-miR-99a | 1 | MIMAT0006668 | aacccguagauccgaucuugu |

The microRNAs are microRNAs included in the top 20 discriminants selected as discriminants including characteristic and/or frequent microRNAs in hepatocellular cancer after model discriminants for predicting hepatocellular cancer are made by statistical analysis processing using the LASSO regression analysis based on the results of the gene expression analysis of the microRNAs extracted from the dog suffering from hepatocellular cancer and the healthy dog. The discriminants and the AUCs indicating the discrimination abilities thereof are shown in FIG. 4 ("healthy vs hepatocellular cancer").

In the diagnostic step or the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, 22, 23, all the 24 of the microRNAs are determined, and the degree of the risk of being suffering from hepatocellular cancer can be diagnosed based on the gene expression levels.

Both of microRNA at a particularly high gene expression level and microRNA at a particularly low gene expression level can be included in the "specific microRNA" as compared with the healthy dog in hepatocellular cancer.

Accordingly, it is desirable in the diagnosis to diagnose a dog having a high gene expression level of at least one microRNA selected from the group consisting of, for example, cfa-miR-122, cfa-miR-206, cfa-miR-378, cfa-miR-483, cfa-miR-665, cfa-miR-718, cfa-miR-8794, cfa-miR-8798, cfa-miR-8875, cfa-miR-8908a-3p, and cfa-miR-92b as being at a high risk of being suffering from hepatocellular cancer, and/or to diagnose a dog having a high gene expression level of at least one microRNA selected from the group consisting of cfa-let-7g, cfa-miR-10a, cfa-miR-125b, cfa-miR-150, cfa-miR-155, cfa-miR-199, cfa-miR-383, cfa-miR-7, cfa-miR-8900, cfa-miR-8902, cfa-miR-8907, cfa-miR-8908d, and cfa-miR-99a as being at a low risk of being suffering from hepatocellular cancer.

It is believed that the diagnosis of canine cancer using the "specific microRNA" as a biomarker is required for the monitoring of the recurrence of hepatocellular cancer after the treatment thereof, and the like besides cancer screening or the like.

### <microRNA to use for diagnosing the mastocytoma>

Examples of a biomarker that is effective for distinguishing the diagnosis of a dog suffering from mastocytoma from a healthy dog include 17 microRNAs shown below:
cfa-miR-10b, cfa-miR-1185, cfa-miR-125b, cfa-miR-126, cfa-miR-149, cfa-miR-150, cfa-miR-155, cfa-miR-197, cfa-miR-199, cfa-miR-222, cfa-miR-24, cfa-miR-26b, cfa-miR-8803, cfa-miR-8872, cfa-miR-8903, cfa-miR-8907 and cfa-miR-92b.
The frequencies and the nucleotide sequences of the microRNAs are shown below.

**[Table 6]**

| miRNA | Frequenc y | Accession No. | Seq |
|---|---|---|---|
| cfa-miR-10b | 1 | MIMAT0009837 | cccuguagaaccgaauuugugu |
| cfa-miR-1185 | 8 | MIMAT0034383 | auauacagggggagacucuuau |
| cfa-miR-125b | 2 | MIMAT0006670 | ucccugagacccuaacuuguga |
| cfa-miR-126 | 1 | MIMAT0006730 | cauuauuacuuuugguacgcg |
| cfa-miR-149 | 20 | MIMAT0009884 | ucuggcuccgugucuucacuccc |
| cfa-miR-150 | 20 | MIMAT0006602 | ucucccaacccuuguaccagug |
| cfa-miR-155 | 11 | MIMAT0006671 | uuaaugcuaaucgugauaggggu |
| cfa-miR-197 | 3 | MIMAT0006698 | uucaccaccuucuccacccagc |
| cfa-miR-199 | 1 | MIMAT0006642 | acaguagucugcacauugguu |
| cfa-miR-222 | 8 | MIMAT0009851 | agcuacaucuggcuacugggu |
| cfa-miR-24 | 8 | MIMAT0006614 | uggcucaguucagcaggaacagg |
| cfa-miR-26b | 1 | MIMAT0006678 | uucaaguaauucaggauagguu |
| cfa-miR-8803 | 5 | MIMAT0034293 | cucagccccugauucucuagc |
| cfa-miR-8872 | 6 | MIMAT0034369 | uucuggguuguggcguccgaggagc |
| cfa-miR-8903 | 2 | MIMAT0034414 | ucuugggccccacccccggagacu |
| cfa-miR-8907 | 17 | MIMAT0034435 | ugccgauucugaagugggaaga |
| cfa-miR-92b | 20 | MIMAT0006703 | uauugcacucgucccggccucc |

The microRNAs are microRNAs included in the top 20 discriminants selected as discriminants including characteristic and/or frequent microRNAs in hepatocellular cancer after model discriminants for predicting mastocytoma are made by statistical analysis processing using the LASSO regression analysis based on the results of the gene expression analysis of the microRNAs extracted from the dog suffering from mastocytoma and the healthy dog. The discriminants and the AUCs indicating the discrimination abilities thereof are shown in FIG. 4 ("healthy vs mastocytoma").

According to the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, or all the 17 of the microRNAs are determined, and the degree of the risk of being suffering from mastocytoma can be diagnosed based on the gene expression levels.

Both of microRNA at a particularly high gene expression level and microRNA at a particularly low gene expression level can be included in the "specific microRNA" as compared with the healthy dog in mastocytoma.

Accordingly, it is desirable in the diagnosis to diagnose a dog having a high gene expression level of at least one microRNA selected from the group consisting of cfa-miR-8803, cfa-miR-8903, and cfa-miR-92b as being at a high risk of being suffering from mastocytoma, and/or to diagnose a dog having a high gene expression level of at least one microRNA selected from the group consisting of cfa-miR-126, cfa-miR-150, cfa-miR-155, cfa-miR-199, cfa-miR-8872, and cfa-miR-8907 as being at a low risk of being suffering from mastocytoma.

It is believed that the diagnosis of canine cancer using the "specific microRNA" is required for the monitoring of the recurrence of mastocytoma after the treatment thereof, and the like besides cancer screening or the like.

### <microRNA for diagnosing intraoral melanoma (including distinction between intraoral melanoma and other cancer diseases)>

Examples of a biomarker that is effective for distinguishing a dog suffering from intraoral melanoma from a dog suffering from other cancer diseases and a healthy dog include 35 microRNAs shown below:
cfa-miR-1185, cfa-miR-122, cfa-miR-126, cfa-miR-1271, cfa-miR-1306, cfa-miR-130b, cfa-miR-140, cfa-miR-144, cfa-miR-146a, cfa-miR-150, cfa-miR-155, cfa-miR-192, cfa-miR-197, cfa-miR-222, cfa-miR-29a, cfa-miR-29b, cfa-miR-30b, cfa-miR-370, cfa-miR-378, cfa-miR-486, cfa-miR-486-3p, cfa-miR-489, cfa-miR-494, cfa-miR-532, cfa-miR-551b, cfa-miR-660, cfa-miR-8803, cfa-miR-8816, cfa-miR-8824, cfa-miR-8834b, cfa-miR-8859a, cfa-miR-8875, cfa-miR-8907, cfa-miR-8908d and cfa-miR-92b.

The frequencies and the nucleotide sequences of the microRNAs are shown below.

**[Table 7]**

| miRNA | Frequenc y | Accession No. | Seq |
|---|---|---|---|
| cfa-miR-1185 | 2 | MIMAT0034383 | auauacagggggagacucuuau |
| cfa-miR-122 | 14 | MIMAT0006619 | uggagugugacaaugguguuug |
| cfa-miR-126 | 20 | MIMAT0006730 | cauuauuacuuuugguacgcg |
| cfa-miR-1271 | 1 | MIMAT0006685 | cuuggcaccuaguaagcacu |
| cfa-miR-1306 | 1 | MIMAT0006661 | ccaccuccccugcaaacgucc |
| cfa-miR-130b | 12 | MIMAT0006659 | cagugcaaugaugaaagggcau |
| cfa-miR-140 | 6 | MIMAT0006689 | accacaggguagaaccacgga |
| cfa-miR-144 | 1 | MIMAT0006734 | uacaguauagaugauguacuag |
| cfa-miR-146a | 13 | MIMAT0006684 | ugagaacugaauuccauggguu |
| cfa-miR-150 | 1 | MIMAT0006602 | ucucccaacccuuguaccagug |
| cfa-miR-155 | 1 | MIMAT0006671 | uuaaugcuaaucgugauaggggu |
| cfa-miR-192 | 1 | MIMAT0006632 | cugaccuaugaauugacagcc |
| cfa-miR-197 | 8 | MIMAT0006698 | uucaccaccuucuccacccagc |
| cfa-miR-222 | 17 | MIMAT0009851 | agcuacaucuggcuacugggu |
| cfa-miR-29a | 3 | MIMAT0006626 | uagcaccaucugaaaucgguua |
| cfa-miR-29b | 20 | MIMAT0006625 | uagcaccauuugaaaucaguguu |
| cfa-miR-30b | 1 | MIMAT0006617 | uguaaacauccuacacucagcu |
| cfa-miR-370 | 5 | MIMAT0009889 | gccugcugggguggaaccuggu |
| cfa-miR-378 | 1 | MIMAT0006683 | acuggacuuggagucagaaggc |
| cfa-miR-486 | 3 | MIMAT0011132 | uccuguacugagcugccccga |
| cfa-miR-486-3p | 3 | MIMAT0032036 | ucggggcagcucaguacaggau |
| cfa-miR-489 | 1 | MIMAT0009860 | gugacaucacauauacggcggc |
| cfa-miR-494 | 9 | MIMAT0009905 | ugaaacauacacgggaaaccuc |
| cfa-miR-532 | 13 | MIMAT0006758 | caugccuugaguguaggaccgu |
| cfa-miR-551b | 1 | MIMAT0009913 | gcgacccauacuugguuucag |
| cfa-miR-660 | 8 | MIMAT0006760 | uacccauugcauaucggaguug |
| cfa-miR-8803 | 1 | MIMAT0034293 | cucagccccugauucucuagc |
| cfa-miR-8816 | 20 | MIMAT0034306 | uagaauccaggucaugugacuccc |
| cfa-miR-8824 | 1 | MIMAT0034314 | guuuccaucuccacccccggca |
| cfa-miR-8834b | 3 | MIMAT0034431 | uggaugcucagucagcgggggugcu |
| cfa-miR-8859a | 1 | MIMAT0034354 | uggaucggagccgggguccgga |
| cfa-miR-8875 | 5 | MIMAT0034375 | ugcuguagcggaacccggggcgggc |
| cfa-miR-8907 | 12 | MIMAT0034435 | ugccgauucugaagugggaaga |
| cfa-miR-8908d | 19 | MIMAT0034420 | auuagcgccugacugagugggguc |
| cfa-miR-92b | 5 | MIMAT0006703 | uauugcacucgucccggccucc |

The microRNAs are microRNAs included in the top 20 discriminants selected as discriminants including characteristic and/or frequent microRNAs in intraoral melanoma after model discriminants for predicting intraoral melanoma are made by statistical analysis processing using the LASSO regression analysis based on the results of the gene expression analysis of the microRNAs extracted from the dog suffering from intraoral melanoma, and the dog suffering from other cancer diseases and the healthy dog. The discriminants and the AUCs indicating the discrimination abilities thereof are shown in FIG. 4 ("intraoral melanoma vs others").

According to the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or all the 35 of the microRNAs are determined, and the degree of the risk of being suffering from intraoral melanoma can be diagnosed based on the gene expression levels. In this diagnosis, the determination of whether the risk of being suffering from intraoral melanoma is higher or lower than the risk of being suffering from other cancer diseases, for example, any of urothelial cancer, malignant lymphoma, hepatocellular cancer, and mastocytoma may be included.

It is believed that since the diagnosis of a dog using the "specific microRNA" as a biomarker also enables discriminating intraoral melanoma from other cancer diseases, the diagnosis is supposed to be the most difficult, and is however required to a certain degree.

### <microRNA for diagnosing urothelial cancer (including the distinction between urothelial cancer and other cancer diseases)>

Examples of a biomarker that is effective for distinguishing a dog suffering from urothelial cancer from a dog suffering from other cancer diseases and a healthy dog include 43 microRNAs shown below:
cfa-let-7b, cfa-miR-107, cfa-miR-10b, cfa-miR-122, cfa-miR-125b, cfa-miR-1306, cfa-miR-130a, cfa-miR-133b, cfa-miR-144, cfa-miR-146a, cfa-miR-149, cfa-miR-181a, cfa-miR-185, cfa-miR-193a, cfa-miR-197, cfa-miR-19b, cfa-miR-23a, cfa-miR-29c, cfa-miR-345, cfa-miR-361, cfa-miR-370, cfa-miR-378, cfa-miR-425, cfa-miR-486, cfa-miR-486-3p, cfa-miR-494, cfa-miR-532, cfa-miR-551b, cfa-miR-660, cfa-miR-8794, cfa-miR-8803, cfa-miR-8816, cfa-miR-8834b, cfa-miR-8860, cfa-miR-8873a, cfa-miR-8891, cfa-miR-8903, cfa-miR-8904b, cfa-miR-8907, cfa-miR-8908d, cfa-miR-92b, cfa-miR-93 and cfa-miR-99a.

The frequencies and the nucleotide sequences of the microRNAs are shown below.

**[Table 8]**

| miRNA | Frequenc y | Accession No. | Seq |
|---|---|---|---|
| cfa-let-7b | 3 | MIMAT0009836 | ugagguaguagguugugugguu |
| cfa-miR-107 | 6 | MIMAT0006666 | agcagcauuguacagggcuau |
| cfa-miR-10b | 2 | MIMAT0009837 | cccuguagaaccgaauuugugu |
| cfa-miR-122 | 17 | MIMAT0006619 | uggagugugacaaugguguuug |
| cfa-miR-125b | 10 | MIMAT0006670 | ucccugagacccuaacuuguga |
| cfa-miR-1306 | 1 | MIMAT0006661 | ccaccuccccugcaaacgucc |
| cfa-miR-130a | 3 | MIMAT0006631 | cagugcaauguuaaaagggcau |
| cfa-miR-133b | 12 | MIMAT0009835 | uuugguccccuucaaccagcua |
| cfa-miR-144 | 1 | MIMAT0006734 | uacaguauagaugauguacuag |
| cfa-miR-146a | 1 | MIMAT0006684 | ugagaacugaauuccauggguu |
| cfa-miR-149 | 19 | MIMAT0009884 | ucuggcuccgugucuucacuccc |
| cfa-miR-181a | 1 | MIMAT0006707 | aacauucaacgcugucggugag |
| cfa-miR-185 | 2 | MIMAT0006660 | uggagagaaaggcaguuccuga |
| cfa-miR-193a | 2 | MIMAT0006735 | ugggucuuugcgggcgagauga |
| cfa-miR-197 | 19 | MIMAT0006698 | uucaccaccuucuccacccagc |
| cfa-miR-19b | 9 | MIMAT0006652 | ugugcaaauccaugcaaaacug |
| cfa-miR-23a | 16 | MIMAT0006640 | aucacauugccagggauuu |
| cfa-miR-29c | 2 | MIMAT0006705 | uagcaccauuugaaaucgguua |
| cfa-miR-345 | 2 | MIMAT0006710 | ccugaacuaggggucuggagg |
| cfa-miR-361 | 7 | MIMAT0006751 | uuaucagaaucuccagggguac |
| cfa-miR-370 | 20 | MIMAT0009889 | gccugcugggguggaaccuggu |
| cfa-miR-378 | 20 | MIMAT0006683 | acuggacuuggagucagaaggc |
| cfa-miR-425 | 11 | MIMAT0006639 | aaugacacgaucacucccguuga |
| cfa-miR-486 | 9 | MIMAT0011132 | uccuguacugagcugccccga |
| cfa-miR-486-3p | 13 | MIMAT0032036 | ucggggcagcucaguacaggau |
| cfa-miR-494 | 3 | MIMAT0009905 | ugaaacauacacgggaaaccuc |
| cfa-miR-532 | 12 | MIMAT0006758 | caugccuugaguguaggaccgu |
| cfa-miR-551b | 2 | MIMAT0009913 | gcgacccauacuugguuucag |
| cfa-miR-660 | 1 | MIMAT0006760 | uacccauugcauaucggaguug |
| cfa-miR-8794 | 1 | MIMAT0034284 | ugccccaucaucagccuccccagu |
| cfa-miR-8803 | 1 | MIMAT0034293 | cucagccccugauucucuagc |
| cfa-miR-8816 | 3 | MIMAT0034306 | uagaauccaggucaugugacuccc |
| cfa-miR-8834b | 20 | MIMAT0034431 | uggaugcucagucagcgggggugc u |
| cfa-miR-8860 | 1 | MIMAT0034355 | uacacuagguuuggaggaaagugg g |
| cfa-miR-8873a | 1 | MIMAT0034373 | uccugaaggcagugggguguagc |
| cfa-miR-8891 | 2 | MIMAT0034398 | uacccaguuucggggucgccuggu |
| cfa-miR-8903 | 12 | MIMAT0034414 | ucuugggccccacccccggagacu |
| cfa-miR-8904b | 12 | MIMAT0034424 | uaacagcaccugcgccccggggaga |
| cfa-miR-8907 | 20 | MIMAT0034435 | ugccgauucugaagugggaaga |
| cfa-miR-8908d | 3 | MIMAT0034420 | auuagcgccugacugagugggguc |
| cfa-miR-92b | 1 | MIMAT0006703 | uauugcacucgucccggccucc |
| cfa-miR-93 | 1 | MIMAT0006696 | caaagugcuguucgugcagguag |
| cfa-miR-99a | 5 | MIMAT0006668 | aacccguagauccgaucuugu |

The microRNAs are microRNAs included in the top 20 discriminants selected as discriminants including characteristic and/or frequent microRNAs in urothelial cancer after model discriminants for predicting urothelial cancer are made by statistical analysis processing using the LASSO regression analysis based on the results of the gene expression analysis of the microRNAs extracted from the dog suffering from urothelial cancer, and the dog suffering from other cancer diseases and the healthy dog. The discriminants and the AUCs indicating the discrimination abilities thereof are shown in FIG. 4 ("urothelial cancer vs others").

According to the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, or all the 43 of the microRNAs are determined, and the degree of the risk of being suffering from urothelial cancer can be diagnosed based on the gene expression levels. In this diagnosis, the determination of whether the risk of being suffering from urothelial cancer is higher or lower than the risk of being suffering from other cancer diseases, for example, any of intraoral melanoma, malignant lymphoma, hepatocellular cancer, and mastocytoma may be included.

It is believed that since the diagnosis of canine cancer using the "specific microRNA" as a biomarker also enables discriminating urothelial cancer from other cancer diseases, the diagnosis is supposed to be the most difficult, and is however required to a certain degree.

### <microRNA for diagnosing malignant lymphoma (including the distinction between malignant lymphoma and other cancer diseases)>

Examples of a biomarker that is effective for distinguishing a dog suffering from malignant lymphoma from a dog suffering from other cancer diseases and a healthy dog include 42 microRNAs shown below:
cfa-let-7f, cfa-miR-122, cfa-miR-1306, cfa-miR-130a, cfa-miR-140, cfa-miR-144, cfa-miR-146a, cfa-miR-151, cfa-miR-15a, cfa-miR-181a, cfa-miR-1844, cfa-miR-188, cfa-miR-192, cfa-miR-193a, cfa-miR-21, cfa-miR-22, cfa-miR-23b, cfa-miR-24, cfa-miR-26a, cfa-miR-26b, cfa-miR-27b, cfa-miR-301a, cfa-miR-339, cfa-miR-342, cfa-miR-345, cfa-miR-378, cfa-miR-486, cfa-miR-486-3p, cfa-miR-551b, cfa-miR-574, cfa-miR-631, cfa-miR-8794, cfa-miR-8803, cfa-miR-8809, cfa-miR-8836, cfa-miR-8843, cfa-miR-8863, cfa-miR-8872, cfa-miR-8904b, cfa-miR-8906, cfa-miR-8907 and cfa-miR-8908d.

The frequencies and the nucleotide sequences of the microRNAs are shown below.

**[Table 9]**

| miRNA | Frequenc y | Accession No. | Seq |
|---|---|---|---|
| cfa-let-7f | 1 | MIMAT0006610 | ugagguaguagauuguauaguu |
| cfa-miR-122 | 4 | MIMAT0006619 | uggagugugacaaugguguuug |
| cfa-miR-1306 | 7 | MIMAT0006661 | ccaccuccccugcaaacgucc |
| cfa-miR-130a | 11 | MIMAT0006631 | cagugcaauguuaaaagggcau |
| cfa-miR-140 | 2 | MIMAT0006689 | accacaggguagaaccacgga |
| cfa-miR-144 | 15 | MIMAT0006734 | uacaguauagaugauguacuag |
| cfa-miR-146a | 4 | MIMAT0006684 | ugagaacugaauuccauggguu |
| cfa-miR-151 | 17 | MIMAT0006615 | ucgaggagcucacagucuagu |
| cfa-miR-15a | 2 | MIMAT0006647 | uagcagcacauaaugguuugu |
| cfa-miR-181a | 1 | MIMAT0006707 | aacauucaacgcugucggugag |
| cfa-miR-1844 | 11 | MIMAT0006740 | aggacuacggacgggcugag |
| cfa-miR-188 | 1 | MIMAT0009880 | caucccuugcaugguggagggu |
| cfa-miR-192 | 1 | MIMAT0006632 | cugaccuaugaauugacagcc |
| cfa-miR-193a | 20 | MIMAT0006735 | ugggucuuugcgggcgagauga |
| cfa-miR-21 | 3 | MIMAT0006741 | uagcuuaucagacugauguuga |
| cfa-miR-22 | 1 | MIMAT0006733 | aagcugccaguugaagaacugu |
| cfa-miR-23b | 10 | MIMAT0006612 | aucacauugccagggauua |
| cfa-miR-24 | 6 | MIMAT0006614 | uggcucaguucagcaggaacagg |
| cfa-miR-26a | 1 | MIMAT0006595 | uucaaguaauccaggauaggcu |
| cfa-miR-26b | 1 | MIMAT0006678 | uucaaguaauucaggauagguu |
| cfa-miR-27b | 1 | MIMAT0006613 | uucacaguggcuaaguucugc |
| cfa-miR-301a | 1 | MIMAT0009853 | cagugcaauaguauugucaaagc |
| cfa-miR-339 | 4 | MIMAT0011134 | ucccuguccuccaggagcu |
| cfa-miR-342 | 13 | MIMAT0006709 | ucucacacagaaaucgcacccgu |
| cfa-miR-345 | 2 | MIMAT0006710 | ccugaacuaggggucuggagg |
| cfa-miR-378 | 20 | MIMAT0006683 | acuggacuuggagucagaaggc |
| cfa-miR-486 | 6 | MIMAT0011132 | uccuguacugagcugccccga |
| cfa-miR-486-3p | 8 | MIMAT0032036 | ucggggcagcucaguacaggau |
| cfa-miR-551b | 3 | MIMAT0009913 | gcgacccauacuugguuucag |
| cfa-miR-574 | 10 | MIMAT0006673 | cacgcucaugcacacacccaca |
| cfa-miR-631 | 11 | MIMAT0009922 | gaccuggcccagaccucagc |
| cfa-miR-8794 | 19 | MIMAT0034284 | ugccccaucaucagccuccccagu |
| cfa-miR-8803 | 1 | MIMAT0034293 | cucagccccugauucucuagc |
| cfa-miR-8809 | 6 | MIMAT0034299 | ucauuggcugagcccggaaccgggg |
| cfa-miR-8836 | 3 | MIMAT0034329 | aggaaaggagaagggccaca |
| cfa-miR-8843 | 13 | MIMAT0034336 | uuguuuuuuucucucgccccgccug |
| cfa-miR-8863 | 7 | MIMAT0034360 | uccagaccaccugggaguuggagc |
| cfa-miR-8872 | 1 | MIMAT0034369 | uucuggguuguggcguccgaggagc |
| cfa-miR-8904b | 14 | MIMAT0034424 | uaacagcaccugcgccccggggaga |
| cfa-miR-8906 | 3 | MIMAT0034434 | uccucugcauuuggcugggacggca |
| cfa-miR-8907 | 4 | MIMAT0034435 | ugccgauucugaagugggaaga |
| cfa-miR-8908d | 11 | MIMAT0034420 | auuagcgccugacugagugggguc |

The microRNAs are microRNAs included in the top 20 discriminants selected as discriminants including characteristic and/or frequent microRNAs in malignant lymphoma after model discriminants for predicting malignant lymphoma are made by statistical analysis processing using the LASSO regression analysis based on the results of the gene expression analysis of the microRNAs extracted from the dog suffering from malignant lymphoma, and the dog suffering from other cancer diseases and the healthy dog. The discriminants and the AUCs indicating the discrimination abilities thereof are shown in FIG. 4 ("malignant lymphoma vs others").

According to the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, or all the 42 of the microRNAs are determined, and the degree of the risk of being suffering from malignant lymphoma can be diagnosed based on the gene expression levels. In this diagnosis, the determination of whether the risk of being suffering from malignant lymphoma is higher or lower than the risk of being suffering from other cancer diseases, for example, any of intraoral melanoma, urothelial cancer, hepatocellular cancer, and mastocytoma may be included.

It is believed that since the diagnosis of canine cancer using the "specific microRNA" as a biomarker also enables discriminating malignant lymphoma from other cancer diseases, the diagnosis is supposed to be the most difficult, and is however required to a certain degree.

### <microRNA for diagnosing hepatocellular cancer (including the distinction between hepatocellular cancer and other cancer diseases)>

Examples of a biomarker that is effective for distinguishing a dog suffering from hepatocellular cancer from a dog suffering from other cancer diseases and a healthy dog include 40 microRNAs shown below:
cfa-let-7b, cfa-let-7c, cfa-let-7f, cfa-let-7g, cfa-miR-10b, cfa-miR-122, cfa-miR-1249, cfa-miR-132, cfa-miR-134, cfa-miR-146a, cfa-miR-150, cfa-miR-155, cfa-miR-16, cfa-miR-206, cfa-miR-22, cfa-miR-223, cfa-miR-29a, cfa-miR-30d, cfa-miR-331, cfa-miR-378, cfa-miR-425, cfa-miR-483, cfa-miR-486-3p, cfa-miR-494, cfa-miR-532, cfa-miR-574, cfa-miR-8794, cfa-miR-8815, cfa-miR-8824, cfa-miR-8834b, cfa-miR-8836, cfa-miR-8843, cfa-miR-8860, cfa-miR-8892, cfa-miR-8900, cfa-miR-8903, cfa-miR-8908a-3p, cfa-miR-8908d, cfa-miR-92b and cfa-miR-99a.

The frequencies and the nucleotide sequences of the microRNAs are shown below.

**[Table 10]**

| miRNA | Frequenc y | Accession No. | Seq |
|---|---|---|---|
| cfa-let-7b | 1 | MIMAT0009836 | ugagguaguagguugugugguu |
| cfa-let-7c | 2 | MIMAT0006669 | ugagguaguagguuguaugguu |
| cfa-let-7f | 10 | MIMAT0006610 | ugagguaguagauuguauaguu |
| cfa-let-7g | 1 | MIMAT0006637 | ugagguaguaguuuguacaguu |
| cfa-miR-10b | 2 | MIMAT0009837 | cccuguagaaccgaauuugugu |
| cfa-miR-122 | 20 | MIMAT0006619 | uggagugugacaaugguguuug |
| cfa-miR-1249 | 2 | MIMAT0034321 | acgcccuucccccccuucuuca |
| cfa-miR-132 | 6 | MIMAT0006732 | uaacagucuacagccauggucgc |
| cfa-miR-134 | 3 | MIMAT0009883 | ugugacugguugaccagagggg |
| cfa-miR-146a | 4 | MIMAT0006684 | ugagaacugaauuccauggguu |
| cfa-miR-150 | 20 | MIMAT0006602 | ucucccaacccuuguaccagug |
| cfa-miR-155 | 1 | MIMAT0006671 | uuaaugcuaaucgugauaggggu |
| cfa-miR-16 | 1 | MIMAT0006648 | uagcagcacguaaauauuggcg |
| cfa-miR-206 | 20 | MIMAT0006606 | uggaauguaaggaagugugugg |
| cfa-miR-22 | 19 | MIMAT0006733 | aagcugccaguugaagaacugu |
| cfa-miR-223 | 1 | MIMAT0009852 | ugucaguuugucaaauacccc |
| cfa-miR-29a | 2 | MIMAT0006626 | uagcaccaucugaaaucgguua |
| cfa-miR-30d | 3 | MIMAT0006616 | uguaaacauccccgacuggaagcu |
| cfa-miR-331 | 1 | MIMAT0009895 | gccccugggccuauccuagaa |
| cfa-miR-378 | 3 | MIMAT0006683 | acuggacuuggagucagaaggc |
| cfa-miR-425 | 1 | MIMAT0006639 | aaugacacgaucacucccguuga |
| cfa-miR-483 | 20 | MIMAT0009901 | ucacuccuccccucccgucuu |
| cfa-miR-486-3p | 1 | MIMAT0032036 | ucggggcagcucaguacaggau |
| cfa-miR-494 | 3 | MIMAT0009905 | ugaaacauacacgggaaaccuc |
| cfa-miR-532 | 9 | MIMAT0006758 | caugccuugaguguaggaccgu |
| cfa-miR-574 | 4 | MIMAT0006673 | cacgcucaugcacacacccaca |
| cfa-miR-8794 | 10 | MIMAT0034284 | ugccccaucaucagccuccccagu |
| cfa-miR-8815 | 2 | MIMAT0034305 | ugccaguggccugcgggggacgg |
| cfa-miR-8824 | 5 | MIMAT0034314 | guuuccaucuccacccccggca |
| cfa-miR-8834b | 9 | MIMAT0034431 | uggaugcucagucagcgggggugcu |
| cfa-miR-8836 | 1 | MIMAT0034329 | aggaaaggagaagggccaca |
| cfa-miR-8843 | 1 | MIMAT0034336 | uuguuuuuuucucucgccccgccug |
| cfa-miR-8860 | 1 | MIMAT0034355 | uacacuagguuuggaggaaaguggg |
| cfa-miR-8892 | 1 | MIMAT0034399 | uagucuguagccccggcccccgaau |
| cfa-miR-8900 | 5 | MIMAT0034410 | uaggacuuuaauggcuggagaga |
| cfa-miR-8903 | 1 | MIMAT0034414 | ucuugggccccacccccggagacu |
| cfa-miR-8908a-3p | 17 | MIMAT0034429 | uaauuaggaccucccugagcggagu |
| cfa-miR-8908d | 6 | MIMAT0034420 | auuagcgccugacugagugggguc |
| cfa-miR-92b | 18 | MIMAT0006703 | uauugcacucgucccggccucc |
| cfa-miR-99a | 1 | MIMAT0006668 | aacccguagauccgaucuugu |

The microRNAs are microRNAs included in the top 20 discriminants selected as discriminants including characteristic and/or frequent microRNAs in hepatocellular cancer after model discriminants for predicting hepatocellular cancer are made by statistical analysis processing using the LASSO regression analysis based on the results of the gene expression analysis of the microRNAs extracted from the dog suffering from hepatocellular cancer, and the dog suffering from other cancer diseases and the healthy dog. The discriminants and the AUCs indicating the discrimination abilities thereof are shown in FIG. 4 ("hepatocellular cancer vs others").

According to the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or all the 40 of the microRNAs are determined, and the degree of the risk of being suffering from hepatocellular cancer can be diagnosed based on the gene expression levels. In this diagnosis, the determination of whether the risk of being suffering from hepatocellular cancer is higher or lower than the risk of being suffering from other cancer diseases, for example, any of intraoral melanoma, urothelial cancer, malignant lymphoma, and mastocytoma may be included.

It is believed that since the diagnosis of canine cancer using the "specific microRNA" as a biomarker also enables discriminating hepatocellular cancer from other cancer diseases, the diagnosis is supposed to be the most difficult, and is however required to a certain degree.

### <microRNA for diagnosing mastocytoma (including the distinction between mastocytoma and other cancer diseases)>

Examples of a biomarker that is effective for distinguishing a dog suffering from mastocytoma from a dog suffering from other cancer diseases and a healthy dog include 29 microRNAs shown below:
cfa-let-7c, cfa-let-7f, cfa-miR-10a, cfa-miR-1306, cfa-miR-130a, cfa-miR-144, cfa-miR-149, cfa-miR-186, cfa-miR-188, cfa-miR-18a, cfa-miR-191, cfa-miR-221, cfa-miR-223, cfa-miR-301a, cfa-miR-30c, cfa-miR-342, cfa-miR-370, cfa-miR-451, cfa-miR-486-3p, cfa-miR-551b, cfa-miR-574, cfa-miR-631, cfa-miR-8809, cfa-miR-8815, cfa-miR-8816, cfa-miR-8834a, cfa-miR-8834b, cfa-miR-8903 and cfa-miR-92b.

The frequencies and the nucleotide sequences of the microRNAs are shown below.

**[Table 11]**

| miRNA | Frequency | Accession No. | Seq |
|---|---|---|---|
| cfa-let-7c | 1 | MIMAT0006669 | ugagguaguagguuguaugguu |
| cfa-let-7f | 9 | MIMAT0006610 | ugagguaguagauuguauaguu |
| cfa-miR-10a | 1 | MIMAT0006737 | uacccuguagauccgaauuugu |
| cfa-miR-1306 | 9 | MIMAT0006661 | ccaccuccccugcaaacgucc |
| cfa-miR-130a | 2 | MIMAT0006631 | cagugcaauguuaaaagggcau |
| cfa-miR-144 | 13 | MIMAT0006734 | uacaguauagaugauguacuag |
| cfa-miR-149 | 1 | MIMAT0009884 | ucuggcuccgugucuucacuccc |
| cfa-miR-186 | 1 | MIMAT0006694 | caaagaauucuccuuuugggcu |
| cfa-miR-188 | 9 | MIMAT0009880 | caucccuugcaugguggagggu |
| cfa-miR-18a | 1 | MIMAT0009832 | uaaggugcaucuagugcagaua |
| cfa-miR-191 | 1 | MIMAT0006638 | caacggaaucccaaaagcagcu |
| cfa-miR-221 | 4 | MIMAT0006757 | agcuacauugucugcuggguuu |
| cfa-miR-223 | 1 | MIMAT0009852 | ugucaguuugucaaauacccc |
| cfa-miR-301a | 9 | MIMAT0009853 | cagugcaauaguauugucaaagc |
| cfa-miR-30c | 1 | MIMAT0006605 | uguaaacauccuacacucucagcu |
| cfa-miR-342 | 7 | MIMAT0006709 | ucucacacagaaaucgcacccgu |
| cfa-miR-370 | 1 | MIMAT0009889 | gccugcugggguggaaccuggu |
| cfa-miR-451 | 1 | MIMAT0009870 | aaaccguuaccauuacugaguu |
| cfa-miR-486-3p | 2 | MIMAT0032036 | ucggggcagcucaguacaggau |
| cfa-miR-551b | 1 | MIMAT0009913 | gcgacccauacuugguuucag |
| cfa-miR-574 | 5 | MIMAT0006673 | cacgcucaugcacacacccaca |
| cfa-miR-631 | 1 | MIMAT0009922 | gaccuggcccagaccucagc |
| cfa-miR-8809 | 18 | MIMAT0034299 | ucauuggcugagcccggaaccgggg |
| cfa-miR-8815 | 14 | MIMAT0034305 | ugccaguggccugcgggggacgg |
| cfa-miR-8816 | 12 | MIMAT0034306 | uagaauccaggucaugugacuccc |
| cfa-miR-8834a | 18 | MIMAT0034327 | ugccgggccuggaggcuccgggg |
| cfa-miR-8834b | 2 | MIMAT0034431 | uggaugcucagucagcgggggugcu |
| cfa-miR-8903 | 12 | MIMAT0034414 | ucuugggccccacccccggagacu |
| cfa-miR-92b | 20 | MIMAT0006703 | uauugcacucgucccggccucc |

The microRNAs are microRNAs included in the top 20 discriminants selected as discriminants including characteristic and/or frequent microRNAs in mastocytoma after model discriminants for predicting mastocytoma are made by statistical analysis processing using the LASSO regression analysis based on the results of the gene expression analysis of the microRNAs extracted from the dog suffering from mastocytoma, and the dog suffering from other cancer diseases and the healthy dog. The discriminants and the AUCs indicating the discrimination abilities thereof are shown in FIG. 4 ("mastocytoma vs others").

According to the present invention, the results of the gene expression analysis of the microRNA extracted from body fluid of the dog to be diagnosed are accordingly acquired newly, the gene expression levels of at least 1, 2, 3, 4, 5, 6, 7, 8, 9, 10, 11, 12, 13, 14, 15, 16, 17, 18, 19, 20, 21, 22, 23, 24, 25, 26, 27, 28, or all the 29 of the microRNAs are determined, and the degree of the risk of being suffering from mastocytoma can be diagnosed based on the gene expression levels. In this diagnosis, the determination of whether the risk of being suffering from mastocytoma is higher or lower than the risk of being suffering from other cancer diseases, for example, any of intraoral melanoma, urothelial cancer, malignant lymphoma, and hepatocellular cancer may be included.

It is believed that since the diagnosis of canine cancer using the "specific microRNA" as a biomarker also enables discriminating mastocytoma from other cancer diseases, the diagnosis is supposed to be the most difficult, and is however required to a certain degree.

### Example

Hereinafter, the present invention will be further specifically described by Examples and the like, but is not limited to these.

### 1. Preparation of serum sample

Blood collection tubes were charged with blood collected from a total of 224 individuals consisting of (A) 40 individuals that were healthy dogs, (B) 40 individuals that were dogs suffering from intraoral melanoma, (C) 40 individuals that were dogs suffering from urothelial cancer, (D) 40 individuals that were dogs suffering from malignant lymphoma, (E) 31 individuals that were dogs suffering from hepatocellular cancer, and (F) 33 individuals that were dogs suffering from mastocytoma, and the blood was left to stand at room temperature for 30 minutes and coagulated. After the confirmation of the coagulation, the blood clots were removed from the tube walls with spatulas or the like to separate serums by centrifugation under the following centrifugation conditions: 1,500 g, 30 minutes, rotational radius: 12 cm, and 3,500 rpm. Then, 0.5 mL of each serum that was the supernatant was dispensed into a freezing tube (Eppendorf SE, DNA LoBind Tubes #0030 108.051) and immediately subjected to cryopreservation at -80°C.

### 2. Extraction of microRNA from serum

microRNA was extracted from the serum by the following procedure:
(1) First, a 2.0-mL tube (Eppendorf SE, DNA LoBind Tubes #0030 120.094) was charged with 200 µl of the serum.
(2) Then, 1 mL (five times the volume of the serum) of a QIAzol Lysis Reagent (QIAGEN, RNase Mini Kit #5546994) was added, and the mixture was stirred with a vortex mixer and then left to stand for 5 minutes.
(3) Next, 200 µl of chloroform was added, and the mixture was stirred with the vortex mixer for 15 seconds.
(4) The mixture was left to stand for 2 minutes, and the mixture was then centrifuged at 12,000 g for 15 minutes with a centrifuge cooled to 4°C.
(5) Subsequently, 600 µl of the supernatant was poured into a new 2.0-mL tube, and 900 µl of 100% ethanol was added to this, followed by suspension.
(6) An RNase Mini spin column (QIAGEN, RNase Mini Kit #5546994) set in a collection tube was charged with 500 µl of the solution mixed in (5), and the solution was further centrifuged at 8,000 g for 15 seconds with the centrifuge warmed to room temperature.
(7) The column eluate was removed.
(8) All the remaining solution mixed in (5) was passed through the spin column by centrifugation in the same way as in (6).
(9) Then, 700 µl of RWT solution was added to the column, followed by centrifugation at 8,000 g for 15 seconds.
(10) Next, 500 µl of RPE solution was added to the column, followed by centrifugation at 8,000 g for 15 seconds.
(11) (10) was repeated once again.
(12) The spin column was set in a new collection tube, followed by centrifugation at 12,000 g for 2 minutes.
(13) The spin column was set in a new 1.5-mL tube, 30 to 50 µL of RNase H₂O of was added, and the mixture was left to stand for 1 minute.
(14) The spin column was centrifuged at 8,000 g for 15 seconds to elute a solution containing RNA.
(15) The solution containing the RNA (column eluate) was collected, poured into the same column, and left to stand for 1 minute.
(16) The spin column was centrifuged at 8,000 g for 1 minute to collect the RNA.

### 3. Measurement of collected RNA

The attached gel was filtered through a spin filter using an Agilent RNA 6000 pico kit (Agilent Technologies Japan, Ltd. #5067-1513). The attached ladder marker was subjected to thermal denaturation treatment at 70°C for 10 minutes beforehand. The RNA to be measured was also subjected to thermal denaturation treatment at 70°C for 2 minutes in the same way. Next, 1 µl of RNA Dye was added to 65 µl of the filtered gel, and the mixture was violently shaken and then centrifuged at room temperature and 13,000 g for 10 minutes. A chip was set in an exclusive holder, the gel was injected, and a condition solution, a fluorescent marker, and the ladder marker were then injected sequentially according to the product manual. Finally, the RNA to be measured was added to the wells for samples on the chip, mixed with a vortex mixier, and the RNA to be measured was measured with an Agilent 2100 Bioanalyzer.

### 4. Microarrays analysis

The microarray analysis was successively performed in the following procedure to detect the expression level of the obtained microRNA.

### (1) Clustering treatment

All the samples were subjected to hierarchical clustering processing based on known 453 microRNAs registered as dog microRNAs, the similarity of the samples were compared, and the samples having expression values close to each other were arranged near to each other. A heat map image shows the height of the gene expression levels of microRNAs, shows that as the color becomes lighter, the gene expression level becomes lower, and shows that as the color becomes darker, the gene expression level becomes higher. In the clustering processing, the Manhattan distance by which the number of genes expressed and differences in the expression level between the genes were emphasized was adopted.

### (2) Principal component analysis

Furthermore, all the 224 samples were subjected to principal component analysis (PCA) to exclude samples having markedly different expression tendencies. As a result of the PCA of all the samples, the first principal component accounted for 30.5%, the second principal component accounted for 9.48%, and the third principal component accounted for 5.88%. Then, 15 samples that were too distant from the other samples were excluded, and the remaining 209 samples were used for subsequent analysis.

### (3) Comparison of expression levels between groups

It was the expression of 121 of the 453 known microRNAs that was detected in the 209 samples. Accordingly, the expression levels of the 121 microRNAs were compared between the group of the dogs suffering from a specific cancer disease and the group of the healthy dogs and further among the group of the dogs suffering from a specific cancer disease, the group of the dogs suffering from other cancer diseases, and the group of the healthy dogs by microarray analysis. The microarray analysis enables obtaining the results of the gene expression analysis of the individual microRNAs such as microRNAs the expression of which increased and microRNAs the expression of which decreased on the contrary in the dogs suffering from the specific cancer disease. The results are shown in FIG. 1.

### 5. Creation of model discriminant

The groups to be discriminated were subjected to the LASSO regression analysis based on the results of the gene expression analysis of the microRNAs to derive model discriminants.

The discriminants were created based on the following three patterns depending on the diagnostic object.

### (1) Pattern A

The specific microRNA that enables discriminating health from the specific cancer disease is selected. It is believed that the diagnosis using such microRNA is required for the monitoring of the recurrence of the specific cancer after the treatment thereof, and the like.

### (2) Pattern B

The specific microRNA that enables discriminating health from the five relatively frequent cancer diseases is selected. It is believed that the diagnosis using such microRNA is useful for inclusively examining whether a dog does not suffer from the five relatively frequent cancer diseases in cancer screening or the like, and is the most highly required.

### (3) Pattern C

The specific microRNA that enables discriminating the specific cancer disease from other cancer diseases and health is selected. It is believed that the diagnosis using such microRNA enables distinguishing the specific cancer disease from not only health but also the other cancers that are difficultly discriminated, and the diagnosis is supposed to be the most difficult, and is however required to a certain degree.

The LASSO regression analysis was specifically performed by the following procedure.
(1) As a data set, the calculating formula representing the microRNA expression that was characteristic between a training group and a validation group wherein training group: validation group = 8:2 was build.
(2) The cross-validation was performed ten times using the training group to create a model.
(3) Evaluated values such as the AUC, the sensitivity, the specificity, the cutoff value, and the like of the model were calculated using the data of the validation group.
(4) The calculation based on the model was repeated 20 times, and the ROC curves in which the sensitivity and the specificity were plotted were made, and the "AUCs" that were areas under the ROC curves were calculated.
(5) The top 20 discriminants were selected by classification in descending order of the "AUCs".

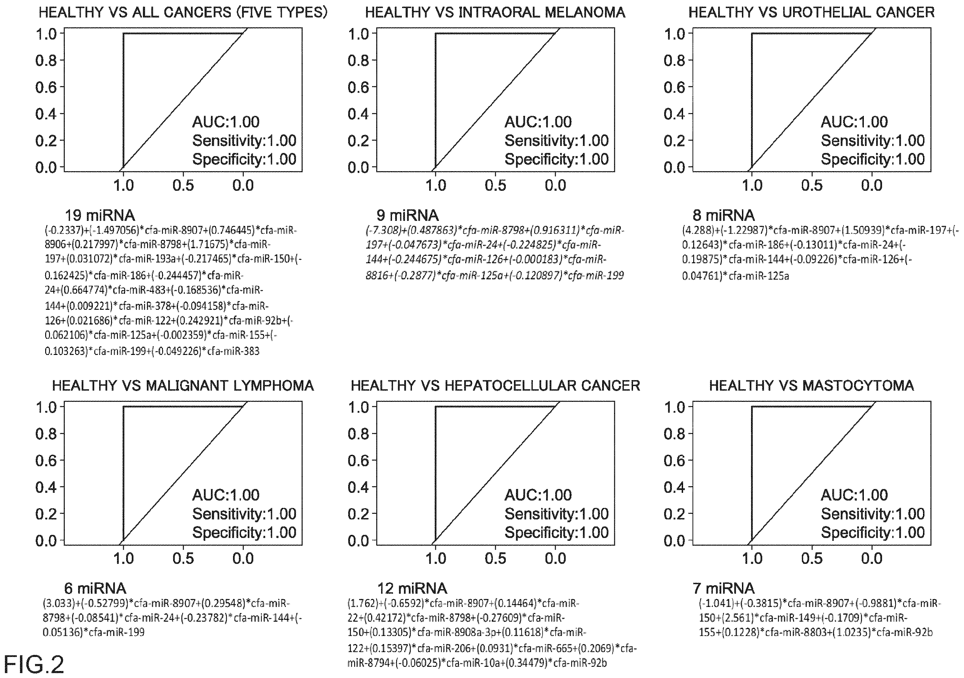

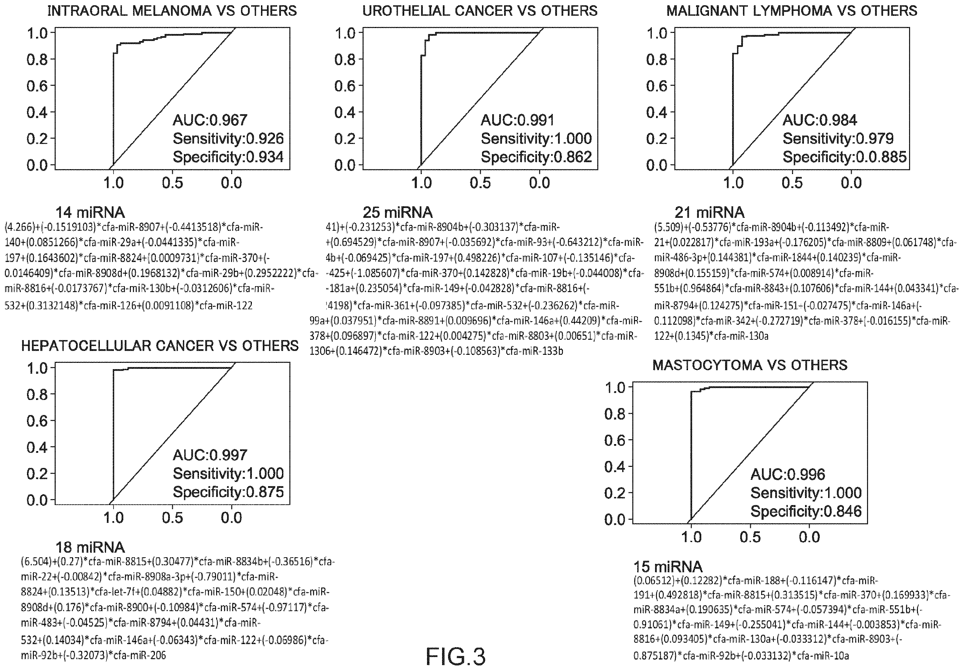

Figures showing the ROC curves and the AUCs of the discriminants created in the LASSO regression analysis are shown in FIGs. 2 and 3. FIGs. 2 and 3 are figures each showing one discriminant as a typical example by selecting the one discriminant for each diagnostic object/use thereof with the vertical axes indicating the sensitivity and the horizontal axes indicating the specificity. It is indicated that as the "AUCs", corresponding to the areas under the ROC curves, become closer to "1", the discrimination abilities become higher. In general, if the "AUCs" are "0.7" or more, it is estimated that the discriminants have discrimination abilities. It is found that, in many of the discriminants in the groups shown in FIG. 2 and corresponding to Pattern A and Pattern B, the "AUCs" reach 1.00 between any groups, and the many have very high discrimination abilities.

It was revealed that as the numbers of the sampled microRNAs increased in the discriminants that corresponded to Pattern C in FIG. 3 and by which the discrimination was supposed to be difficult, the values of the "AUCs" decreased slightly, but most thereof still exhibited high "AUCs" exceeding 0.9, and the microRNAs exhibited enough discrimination abilities when the microRNAs were used as biomarkers.

These results show that the specific microRNA sampled by implementing the present invention exhibited high ability to discriminate the "specific cancer disease", and therefore show that the specific microRNA can be used for diagnosing canine cancer as a biomarker.

### Industrial Applicability

A method for diagnosing canine cancer of the present invention can be used for early diagnosis and early treatment of canine cancer.

Sequence Listing

## Claims

1. A method for diagnosing canine cancer, comprising:
a diagnostic step of acquiring a result of gene expression analysis of microRNA extracted from body fluid of a dog to be diagnosed, determining a gene expression level of a specific microRNA using the result, and diagnosing a degree of a risk of the dog to be diagnosed being suffering from a specific cancer disease using the gene expression level as a diagnostic criterion, **characterized in that** the specific microRNA is a microRNA showing a significant difference in gene expression level between a dog suffering from the specific cancer disease and a healthy dog based on the result of the gene expression analysis of the microRNA extracted from the body fluid of the dog.

2. The method according to claim 1, further comprising a step of sampling the microRNA showing a significant difference in gene expression level between the dog suffering from the specific cancer disease and the healthy dog based on the result of the gene expression analysis of the microRNA extracted from the body fluid of the dog as the specific microRNA on the basis of a discriminant created using statistical analysis processing before the diagnostic step.

3. The method according to claim 1 or 2, wherein the specific cancer disease is selected from the group consisting of intraoral melanoma, urothelial cancer, malignant lymphoma, hepatocellular cancer, and mastocytoma.

4. The method according to any one of claims 1 to 3,
wherein the specific cancer disease is five cancer diseases including intraoral melanoma, urothelial cancer, malignant lymphoma, hepatocellular cancer, and mastocytoma, and
the specific microRNA is at least one selected from the group consisting of cfa-let-7c, cfa-miR-103, cfa-miR-10a, cfa-miR-122, cfa-miR-125a, cfa-miR-125b, cfa-miR-126, cfa-miR-130a, cfa-miR-144, cfa-miR-150, cfa-miR-155, cfa-miR-186, cfa-miR-193a, cfa-miR-197, cfa-miR-199, cfa-miR-19b, cfa-miR-22, cfa-miR-222, cfa-miR-223, cfa-miR-24, cfa-miR-26a, cfa-miR-27b, cfa-miR-339, cfa-miR-342, cfa-miR-378, cfa-miR-383, cfa-miR-483, cfa-miR-486-3p, cfa-miR-489, cfa-miR-551b, cfa-miR-660, cfa-miR-718, cfa-miR-874, cfa-miR-8794, cfa-miR-8798, cfa-miR-8843, cfa-miR-8859a, cfa-miR-8860, cfa-miR-8903, cfa-miR-8906, cfa-miR-8907, cfa-miR-8908a-3p, cfa-miR-8908d and cfa-miR-92b.

5. The method according to any one of claims 1 to 3,
wherein the specific cancer disease is intraoral melanoma, and
the specific microRNA is at least one selected from the group consisting of cfa-miR-10a, cfa-miR-1185, cfa-miR-125a, cfa-miR-126, cfa-miR-144, cfa-miR-146a, cfa-miR-149, cfa-miR-150, cfa-miR-155, cfa-miR-184, cfa-miR-186, cfa-miR-197, cfa-miR-199, cfa-miR-19b, cfa-miR-24, cfa-miR-30c, cfa-miR-483, cfa-miR-489, cfa-miR-8798, cfa-miR-8816, cfa-miR-8875 and cfa-miR-8908a-3p.

6. The method according to any one of claims 1 to 3,
wherein the specific cancer disease is urothelial cancer, and
the specific microRNA is at least one selected from the group consisting of cfa-let-7f, cfa-miR-10a, cfa-miR-1249, cfa-miR-125a, cfa-miR-126, cfa-miR-1306, cfa-miR-130a, cfa-miR-133b, cfa-miR-144, cfa-miR-146a, cfa-miR-149, cfa-miR-150, cfa-miR-155, cfa-miR-1844, cfa-miR-186, cfa-miR-193a, cfa-miR-197, cfa-miR-19b, cfa-miR-2114, cfa-miR-24, cfa-miR-29b, cfa-miR-339, cfa-miR-342, cfa-miR-361, cfa-miR-370, cfa-miR-425, cfa-miR-483, cfa-miR-494, cfa-miR-502, cfa-miR-551b, cfa-miR-660, cfa-miR-665, cfa-miR-718, cfa-miR-8798, cfa-miR-8824, cfa-miR-8832, cfa-miR-8834a, cfa-miR-8843, cfa-miR-8904b and cfa-miR-8907, cfa-miR-8908a-3p.

7. The method according to any one of claims 1 to 3,
wherein the specific cancer disease is malignant lymphoma, and
the specific microRNA is at least one selected from the group consisting of cfa-miR-1185, cfa-miR-122, cfa-miR-1249, cfa-miR-126, cfa-miR-1306, cfa-miR-144, cfa-miR-150, cfa-miR-184, cfa-miR-188, cfa-miR-197, cfa-miR-199, cfa-miR-24, cfa-miR-342, cfa-miR-345, cfa-miR-378, cfa-miR-425, cfa-miR-574, cfa-miR-8794, cfa-miR-8797, cfa-miR-8798, cfa-miR-8900, cfa-miR-8904b, cfa-miR-8907 and cfa-miR-8908d.

8. The method according to any one of claims 1 to 3,
wherein the specific cancer disease is hepatocellular cancer, and
the specific microRNA is at least one selected from the group consisting of cfa-let-7g, cfa-miR-10a, cfa-miR-1185, cfa-miR-122, cfa-miR-125b, cfa-miR-150, cfa-miR-155, cfa-miR-197, cfa-miR-199, cfa-miR-206, cfa-miR-22, cfa-miR-378, cfa-miR-383, cfa-miR-483, cfa-miR-665, cfa-miR-7, cfa-miR-718, cfa-miR-874, cfa-miR-8794, cfa-miR-8798, cfa-miR-8875, cfa-miR-8900, cfa-miR-8902, cfa-miR-8907, cfa-miR-8908a-3p, cfa-miR-8908d, cfa-miR-92b and cfa-miR-99a.

9. The method according to any one of claims 1 to 3,
wherein the specific cancer disease is mastocytoma, and
the specific microRNA is at least one selected from the group consisting of cfa-miR-10b, cfa-miR-1185, cfa-miR-125b, cfa-miR-126, cfa-miR-149, cfa-miR-150, cfa-miR-155, cfa-miR-197, cfa-miR-199, cfa-miR-222, cfa-miR-24, cfa-miR-26b, cfa-miR-8803, cfa-miR-8872, cfa-miR-8903, cfa-miR-8907 and cfa-miR-92b.

10. A method for diagnosing canine cancer, comprising:
a diagnostic step of acquiring a result of gene expression analysis of microRNA extracted from body fluid of a dog to be diagnosed, determining a gene expression level of a specific microRNA using the result, and diagnosing a degree of a risk of the dog to be diagnosed being suffering from a specific cancer disease using the gene expression level as a diagnostic criterion, **characterized in that** the specific microRNA is a microRNA showing a significant difference in gene expression level among a dog suffering from the specific cancer disease, a dog suffering from another cancer disease, and a healthy dog based on the result of the gene expression analysis of the microRNA extracted from the body fluid of the dog.

11. The method according to claim 10, further comprising a step of sampling the microRNA showing a significant difference in gene expression level among the dog suffering from the specific cancer disease, the dog suffering from other cancer disease, and the healthy dog based on the result of the gene expression analysis of the microRNA extracted from the body fluid of the dog as the specific microRNA on the basis of a discriminant created using statistical analysis processing before the diagnostic step.

12. The method according to claim 10, comprising:
the degree of the risk of the dog to be diagnosed being suffering from the specific cancer disease being higher or lower than a risk of being suffering from other cancer disease in the diagnostic step.

13. The method according to any one of claims 10 to 12,
wherein the specific cancer disease is selected from the group consisting of intraoral melanoma, urothelial cancer, malignant lymphoma, hepatocellular cancer, and mastocytoma.

14. The method according to any one of claims 10 to 13,
wherein the specific cancer disease is intraoral melanoma, and
the specific microRNA that is used in the diagnosis is at least one selected from the group consisting of cfa-miR-1185, cfa-miR-122, cfa-miR-126, cfa-miR-1271, cfa-miR-1306, cfa-miR-130b, cfa-miR-140, cfa-miR-144, cfa-miR-146a, cfa-miR-150, cfa-miR-155, cfa-miR-192, cfa-miR-197, cfa-miR-222, cfa-miR-29a, cfa-miR-29b, cfa-miR-30b, cfa-miR-370, cfa-miR-378, cfa-miR-486, cfa-miR-486-3p, cfa-miR-489, cfa-miR-494, cfa-miR-532, cfa-miR-551b, cfa-miR-660, cfa-miR-8803, cfa-miR-8816, cfa-miR-8824, cfa-miR-8834b, cfa-miR-8859a, cfa-miR-8875, cfa-miR-8907, cfa-miR-8908d and cfa-miR-92b.

15. The method according to any one of claims 10 to 13,
wherein the specific cancer disease is urothelial cancer, and
the specific microRNA that is used in the diagnosis is at least one selected from the group consisting of cfa-let-7b, cfa-miR-107, cfa-miR-10b, cfa-miR-122, cfa-miR-125b, cfa-miR-1306, cfa-miR-130a, cfa-miR-133b, cfa-miR-144, cfa-miR-146a, cfa-miR-149, cfa-miR-181a, cfa-miR-185, cfa-miR-193a, cfa-miR-197, cfa-miR-19b, cfa-miR-23a, cfa-miR-29c, cfa-miR-345, cfa-miR-361, cfa-miR-370, cfa-miR-378, cfa-miR-425, cfa-miR-486, cfa-miR-486-3p, cfa-miR-494, cfa-miR-532, cfa-miR-551b, cfa-miR-660, cfa-miR-8794, cfa-miR-8803, cfa-miR-8816, cfa-miR-8834b, cfa-miR-8860, cfa-miR-8873a, cfa-miR-8891, cfa-miR-8903, cfa-miR-8904b, cfa-miR-8907, cfa-miR-8908d, cfa-miR-92b, cfa-miR-93 and cfa-miR-99a.

16. The method according to any one of claims 10 to 13,
wherein the specific cancer disease is malignant lymphoma, and
the specific microRNA that is used in the diagnosis is at least one selected from the group consisting of cfa-let-7f, cfa-miR-122, cfa-miR-1306, cfa-miR-130a, cfa-miR-140, cfa-miR-144, cfa-miR-146a, cfa-miR-151, cfa-miR-15a, cfa-miR-181a, cfa-miR-1844, cfa-miR-188, cfa-miR-192, cfa-miR-193a, cfa-miR-21, cfa-miR-22, cfa-miR-23b, cfa-miR-24, cfa-miR-26a, cfa-miR-26b, cfa-miR-27b, cfa-miR-301a, cfa-miR-339, cfa-miR-342, cfa-miR-345, cfa-miR-378, cfa-miR-486, cfa-miR-486-3p, cfa-miR-551b, cfa-miR-574, cfa-miR-631, cfa-miR-8794, cfa-miR-8803, cfa-miR-8809, cfa-miR-8836, cfa-miR-8843, cfa-miR-8863, cfa-miR-8872, cfa-miR-8904b, cfa-miR-8906, cfa-miR-8907 and cfa-miR-8908d.

17. The method according to any one of claims 10 to 13,
wherein the specific cancer disease is hepatocellular cancer, and
the specific microRNA that is used in the diagnosis is at least one selected from the group consisting of cfa-let-7b, cfa-let-7c, cfa-let-7f, cfa-let-7g, cfa-miR-10b, cfa-miR-122, cfa-miR-1249, cfa-miR-132, cfa-miR-134, cfa-miR-146a, cfa-miR-150, cfa-miR-155, cfa-miR-16, cfa-miR-206, cfa-miR-22, cfa-miR-223, cfa-miR-29a, cfa-miR-30d, cfa-miR-331, cfa-miR-378, cfa-miR-425, cfa-miR-483, cfa-miR-486-3p, cfa-miR-494, cfa-miR-532, cfa-miR-574, cfa-miR-8794, cfa-miR-8815, cfa-miR-8824, cfa-miR-8834b, cfa-miR-8836, cfa-miR-8843, cfa-miR-8860, cfa-miR-8892, cfa-miR-8900, cfa-miR-8903, cfa-miR-8908a-3p, cfa-miR-8908d, cfa-miR-92b and cfa-miR-99a.

18. The method according to any one of claims 10 to 13,
wherein the specific cancer disease is mastocytoma, and
the specific microRNA that is used in the diagnosis is at least one selected from the group consisting of cfa-let-7c, cfa-let-7f, cfa-miR-10a, cfa-miR-1306, cfa-miR-130a, cfa-miR-144, cfa-miR-149, cfa-miR-186, cfa-miR-188, cfa-miR-18a, cfa-miR-191, cfa-miR-221, cfa-miR-223, cfa-miR-301a, cfa-miR-30c, cfa-miR-342, cfa-miR-370, cfa-miR-451, cfa-miR-486-3p, cfa-miR-551b, cfa-miR-574, cfa-miR-631, cfa-miR-8809, cfa-miR-8815, cfa-miR-8816, cfa-miR-8834a, cfa-miR-8834b, cfa-miR-8903 and cfa-miR-92b.

19. A biomarker for diagnosing five canine cancer diseases including intraoral melanoma, urothelial cancer, malignant lymphoma, hepatocellular cancer, and mastocytoma, comprising:
at least one microRNA selected from the group consisting of cfa-let-7c, cfa-miR-103, cfa-miR-10a, cfa-miR-122, cfa-miR-125a, cfa-miR-125b, cfa-miR-126, cfa-miR-130a, cfa-miR-144, cfa-miR-150, cfa-miR-155, cfa-miR-186, cfa-miR-193a, cfa-miR-197, cfa-miR-199, cfa-miR-19b, cfa-miR-22, cfa-miR-222, cfa-miR-223, cfa-miR-24, cfa-miR-26a, cfa-miR-27b, cfa-miR-339, cfa-miR-342, cfa-miR-378, cfa-miR-383, cfa-miR-483, cfa-miR-486-3p, cfa-miR-489, cfa-miR-551b, cfa-miR-660, cfa-miR-718, cfa-miR-874, cfa-miR-8794, cfa-miR-8798, cfa-miR-8843, cfa-miR-8859a, cfa-miR-8860, cfa-miR-8903, cfa-miR-8906, cfa-miR-8907, cfa-miR-8908a-3p, cfa-miR-8908d and cfa-miR-92b.

20. A biomarker for diagnosing dog intraoral melanoma, comprising:
at least one of microRNA selected from the group consisting of cfa-miR-10a, cfa-miR-1185, cfa-miR-125a, cfa-miR-126, cfa-miR-144, cfa-miR-146a, cfa-miR-149, cfa-miR-150, cfa-miR-155, cfa-miR-184, cfa-miR-186, cfa-miR-197, cfa-miR-199, cfa-miR-19b, cfa-miR-24, cfa-miR-30c, cfa-miR-483, cfa-miR-489, cfa-miR-8798, cfa-miR-8816, cfa-miR-8875 and cfa-miR-8908a-3p.

21. A biomarker for diagnosing dog urothelial cancer, comprising:
at least one microRNA selected from the group consisting of cfa-let-7f, cfa-miR-10a, cfa-miR-1249, cfa-miR-125a, cfa-miR-126, cfa-miR-1306, cfa-miR-130a, cfa-miR-133b, cfa-miR-144, cfa-miR-146a, cfa-miR-149, cfa-miR-150, cfa-miR-155, cfa-miR-1844, cfa-miR-186, cfa-miR-193a, cfa-miR-197, cfa-miR-19b, cfa-miR-2114, cfa-miR-24, cfa-miR-29b, cfa-miR-339, cfa-miR-342, cfa-miR-361, cfa-miR-370, cfa-miR-425, cfa-miR-483, cfa-miR-494, cfa-miR-502, cfa-miR-551b, cfa-miR-660, cfa-miR-665, cfa-miR-718, cfa-miR-8798, cfa-miR-8824, cfa-miR-8832, cfa-miR-8834a, cfa-miR-8843, cfa-miR-8904b and cfa-miR-8907, cfa-miR-8908a-3p.

22. A biomarker for diagnosing a dog malignant lymphoma, comprising:
at least one microRNA selected from the group consisting of cfa-miR-1185, cfa-miR-122, cfa-miR-1249, cfa-miR-126, cfa-miR-1306, cfa-miR-144, cfa-miR-150, cfa-miR-184, cfa-miR-188, cfa-miR-197, cfa-miR-199, cfa-miR-24, cfa-miR-342, cfa-miR-345, cfa-miR-378, cfa-miR-425, cfa-miR-574, cfa-miR-8794, cfa-miR-8797, cfa-miR-8798, cfa-miR-8900, cfa-miR-8904b, cfa-miR-8907 and cfa-miR-8908d.

23. A biomarker for diagnosing dog hepatocellular cancer, comprising:
at least one the microRNA selected from the group consisting of cfa-let-7g, cfa-miR-10a, cfa-miR-1185, cfa-miR-122, cfa-miR-125b, cfa-miR-150, cfa-miR-155, cfa-miR-197, cfa-miR-199, cfa-miR-206, cfa-miR-22, cfa-miR-378, cfa-miR-383, cfa-miR-483, cfa-miR-665, cfa-miR-7, cfa-miR-718, cfa-miR-874, cfa-miR-8794, cfa-miR-8798, cfa-miR-8875, cfa-miR-8900, cfa-miR-8902, cfa-miR-8907, cfa-miR-8908a-3p, cfa-miR-8908d, cfa-miR-92b and cfa-miR-99a.

24. A biomarker for diagnosing dog mastocytoma, comprising:
at least one microRNA selected from the group consisting of cfa-miR-10b, cfa-miR-1185, cfa-miR-125b, cfa-miR-126, cfa-miR-149, cfa-miR-150, cfa-miR-155, cfa-miR-197, cfa-miR-199, cfa-miR-222, cfa-miR-24, cfa-miR-26b, cfa-miR-8803, cfa-miR-8872, cfa-miR-8903, cfa-miR-8907 and cfa-miR-92b.

25. A biomarker for diagnosing dog intraoral melanoma, comprising:
at least one microRNA selected from the group consisting of cfa-miR-1185, cfa-miR-122, cfa-miR-126, cfa-miR-1271, cfa-miR-1306, cfa-miR-130b, cfa-miR-140, cfa-miR-144, cfa-miR-146a, cfa-miR-150, cfa-miR-155, cfa-miR-192, cfa-miR-197, cfa-miR-222, cfa-miR-29a, cfa-miR-29b, cfa-miR-30b, cfa-miR-370, cfa-miR-378, cfa-miR-486, cfa-miR-486-3p, cfa-miR-489, cfa-miR-494, cfa-miR-532, cfa-miR-551b, cfa-miR-660, cfa-miR-8803, cfa-miR-8816, cfa-miR-8824, cfa-miR-8834b, cfa-miR-8859a, cfa-miR-8875, cfa-miR-8907, cfa-miR-8908d and cfa-miR-92b.

26. A biomarker for diagnosing dog urothelial cancer, comprising:
at least one microRNA selected from the group consisting of cfa-let-7b, cfa-miR-107, cfa-miR-10b, cfa-miR-122, cfa-miR-125b, cfa-miR-1306, cfa-miR-130a, cfa-miR-133b, cfa-miR-144, cfa-miR-146a, cfa-miR-149, cfa-miR-181a, cfa-miR-185, cfa-miR-193a, cfa-miR-197, cfa-miR-19b, cfa-miR-23a, cfa-miR-29c, cfa-miR-345, cfa-miR-361, cfa-miR-370, cfa-miR-378, cfa-miR-425, cfa-miR-486, cfa-miR-486-3p, cfa-miR-494, cfa-miR-532, cfa-miR-551b, cfa-miR-660, cfa-miR-8794, cfa-miR-8803, cfa-miR-8816, cfa-miR-8834b, cfa-miR-8860, cfa-miR-8873a, cfa-miR-8891, cfa-miR-8903, cfa-miR-8904b, cfa-miR-8907, cfa-miR-8908d, cfa-miR-92b, cfa-miR-93 and cfa-miR-99a.

27. A biomarker for diagnosing dog malignant lymphoma, comprising:
at least one microRNA selected from the group consisting of cfa-let-7f, cfa-miR-122, cfa-miR-1306, cfa-miR-130a, cfa-miR-140, cfa-miR-144, cfa-miR-146a, cfa-miR-151, cfa-miR-15a, cfa-miR-181a, cfa-miR-1844, cfa-miR-188, cfa-miR-192, cfa-miR-193a, cfa-miR-21, cfa-miR-22, cfa-miR-23b, cfa-miR-24, cfa-miR-26a, cfa-miR-26b, cfa-miR-27b, cfa-miR-301a, cfa-miR-339, cfa-miR-342, cfa-miR-345, cfa-miR-378, cfa-miR-486, cfa-miR-486-3p, cfa-miR-551b, cfa-miR-574, cfa-miR-631, cfa-miR-8794, cfa-miR-8803, cfa-miR-8809, cfa-miR-8836, cfa-miR-8843, cfa-miR-8863, cfa-miR-8872, cfa-miR-8904b, cfa-miR-8906, cfa-miR-8907 and cfa-miR-8908d.

28. A biomarker for diagnosing dog hepatocellular cancer, comprising:
at least one microRNA selected from the group consisting of cfa-let-7b, cfa-let-7c, cfa-let-7f, cfa-let-7g, cfa-miR-10b, cfa-miR-122, cfa-miR-1249, cfa-miR-132, cfa-miR-134, cfa-miR-146a, cfa-miR-150, cfa-miR-155, cfa-miR-16, cfa-miR-206, cfa-miR-22, cfa-miR-223, cfa-miR-29a, cfa-miR-30d, cfa-miR-331, cfa-miR-378, cfa-miR-425, cfa-miR-483, cfa-miR-486-3p, cfa-miR-494, cfa-miR-532, cfa-miR-574, cfa-miR-8794, cfa-miR-8815, cfa-miR-8824, cfa-miR-8834b, cfa-miR-8836, cfa-miR-8843, cfa-miR-8860, cfa-miR-8892, cfa-miR-8900, cfa-miR-8903, cfa-miR-8908a-3p, cfa-miR-8908d, cfa-miR-92b and cfa-miR-99a.

29. A biomarker for diagnosing dog mastocytoma, comprising:
at least one microRNA selected from the group consisting of cfa-let-7c, cfa-let-7f, cfa-miR-10a, cfa-miR-1306, cfa-miR-130a, cfa-miR-144, cfa-miR-149, cfa-miR-186, cfa-miR-188, cfa-miR-18a, cfa-miR-191, cfa-miR-221, cfa-miR-223, cfa-miR-301a, cfa-miR-30c, cfa-miR-342, cfa-miR-370, cfa-miR-451, cfa-miR-486-3p, cfa-miR-551b, cfa-miR-574, cfa-miR-631, cfa-miR-8809, cfa-miR-8815, cfa-miR-8816, cfa-miR-8834a, cfa-miR-8834b, cfa-miR-8903 and cfa-miR-92b.
